(19) Europäisches Patentamt
European Patent Office
Office européen des brevets

(11) Veröffentlichungsnummer: **0 161 222**
**B1**

(12) # EUROPÄISCHE PATENTSCHRIFT

(45) Veröffentlichungstag der Patentschrift: **10.10.90**

(51) Int. Cl.$^5$: **C 09 D 5/08**

(21) Anmeldenummer: **85810218.9**

(22) Anmeldetag: **08.05.85**

(54) **Heterocylische Mercaptocarbonsäureamide, -imide und -nitrile als Korrosionsinhibitoren.**

(30) Priorität: **11.05.84 GB 8412064**

(43) Veröffentlichungstag der Anmeldung:
**13.11.85 Patentblatt 85/46**

(45) Bekanntmachung des Hinweises auf die Patenterteilung:
**10.10.90 Patentblatt 90/41**

(84) Benannte Vertragsstaaten:
**BE CH DE FR GB IT LI NL**

(56) Entgegenhaltungen:
**CH-A- 458 048**

(73) Patentinhaber: **CIBA-GEIGY AG**
**Klybeckstrasse 141**
**CH-4002 Basel (CH)**

(72) Erfinder: **Clubley, Brian George**
**7 Green Villa Park**
**Wilmslow Cheshire SK9 6EJ (GB)**
Erfinder: **Phillips, Emyr**
**12 Pinewood Court Broad Road**
**Sale Cheshire M33 2ES (GB)**

**EP 0 161 222 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft die Verwendung von heterocyclischen Mercaptocarbonsäureamiden, -imiden und -nitrilen als Korrosionsinhibitoren, die solche Verbindungen enthaltende antikorrosiven Systeme und die Verbindungen selbst, soweit sie neu sind.

Korrosionsschutz ist eine der wichtigsten Aufgaben von organischen Ueberzugsmitteln für Metallsubstrate. Viele Vorschläge, den Korrosionsschutz von Anstrichen zu verbessern, sind in der Literatur zu finden z.B. in H. Kittel, Lehrbuch der Lacke und Beschichtungen, Band V, Stuttgart 1977, 46—103. Einmal kann die Barrierenfunktion des Ueberzugsmittels verbessert werden, um korrosive Agentien wie Sauerstoff, Wasser und Ionen von der Metalloberfläche abzuhalten. Zum anderen können antikorrosive Pigmente zugesetzt werden, welche chemisch oder elektrochemisch in den Korrosionsprozess eingreifen, bespeilsweise durch Bildung unlöslicher Abscheidungen mit Korrosionsprodukten oder durch Passivierung (Polarisierung) der Metalloberfläche. Zu den wirkungsvollsten antikorrosiven Pigmenten zählen Metallchromate und Bleiverbindungen. Metallchromate wurden vor allem deshalb viel verwendet, weil sie sowohl die anodische wie die kathodische Korrosion inhibieren. Heute hat man gewisse Bedenken gegen die Verwendung von Chromaten wegen ihrer potentiellen krebserregenden Wirkung. In ähnlicher Weise hat man gegen die Verwendung von Bleiverbindungen Bedenken wegen ihrer chronischen Toxizität.

Auch Metallsalze von organischen Verbindungen wurden vielfach als Korrosionsinhibitoren vorgeschlagen. So empfiehlt z.B. die Europ. Patentschrift 3817 die Verwendung von Zink- oder Bleisalzen von Hydroxyl- oder Mercaptoverbindungen von 5- oder 6-gliedrigen heterocyclischen Verbindungen, die die charakterische Gruppe

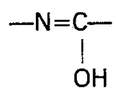

oder

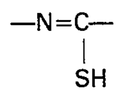

enthalten. Ein typisches Beispiel hierfür sind die Zn- oder Pb-Salze von 2-Mercaptobenzthiazol.

In der CH—A—458 048 sind Derivate von 2-Mercaptooxazol und 2-Mercaptoimidazol beschrieben, die als Korrosionsschutzmittel bei der Herstellung von optisch aufgehelltem Papier verwendbar sind, insbesondere auf Maschinen mit Kupfersieb.

Wie in Farbe und Lack *87* (1981), 787, dargelegt wurde, bestehen in der Fachwelt Zweifel, ob der Zusatz organischer Korrosionsinhibitoren allein (ohne Korrosionsschutz-Pigmente) einen für die Praxis ausreichende Korrosionsschutz auf dem Anstrichsektor gewährleisten kann.

Aehnliche Ueberlegungen gelten für wässrige Systeme, die mit Metallen in Kontakt stehen, wie z.B. Kühl- und Heizwasserkreisläufe, Hydraulikflüssigkeiten oder Metallbearbeitungsflüssigkeiten. Auch hier kann die Korrosion des Metalles durch Zusatz von Korrosionsinhibitoren zum wässrigen Medium vermindert werden, es bestehen jedoch erhebliche ökologische Bedenken wegen der potentiellen Vergiftung der Abwässer.

Es wurde nunmehr gefunden, dass bestimmte heterocyclische Carbonsäureamide, -imide und -nitrile und derent nicht-toxischen Salze wirkungsvolle Korrosionsinhibitoren sind und sowohl in allen Arten von Anstrichstoffen wie in wässrigen Systemen ausserhalb des Anstrichsektors als solche verwendet werden können. Ihr Vorteil ist eine niedrige Toxizität bei hoher Wirksamkeit. Bei der Verwendung in Anstrichstoffen besteht gegenüber korrosionsinhibierenden Pigmenten ferner der Vorteil, dass man frei ist in der Wahl des Farbpigmentes und auch unpigmentierte Klarlacke antikorrosiv formulieren kann.

Die vorliegende Erfindung betrifft die Verwendung von Amiden, Imiden oder Nitrilen von aliphatischen oder cycloaliphatischen Mono-, Di-, Tri- oder Tetracarbonsäuren, die im (cyclo)aliphatischen Rest durch eine oder mehrere Gruppen vorzugsweise nur durch eine Gruppe, der Formel I substituiert sind,

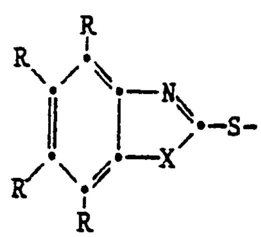

I

worin X Schwefel bedeutet und jedes R unabhängig voneinander Wasserstoff, Alkyl, Halogenalkyl, Alkoxy, Alkylthio, Alkylsulfonyl, Cycloalkyl, Phenyl, Alkyphenyl, Phenylalkyl, Halogen, —CN, —$NO_2$, —COOH, —COOAlkyl, —OH oder eine primäre, sekundäre oder tertiäre Amino- oder Carbamoylgruppe bedeutet, sowie nicht-toxische Basen-Additionssalze dieser Verbindungen, soweit sie Carboxylgruppen enthalten, als Korrosionsinhibitoren.

Unter Amiden von aliphatischen oder cycloaliphatischen Mono-, Di-, Tri- oder Tetracarbonsäuren sind hier sowohl Voll-Amide wie partielle Amide (Halbamide) zu verstehen. Sie enthalten 1 bis 4 Gruppen $—CONZ_1Z_2$ oder —COOH, wobei $Z_1$ und $Z_2$ unabhängig voneinander Wasserstoff, $C_1—C_{18}$-Alkyl, das durch ein oder mehrere O— oder S-Atome oder NR°-Gruppen unterbrochen sein kann (R° = $C_1—C_{18}$-Alkyl, $C_5—C_8$-Cycloalkyl, Phenyl, Naphthyl, $C_7—C_9$-Phenylalkyl oder $C_7—C_{18}$-Alkylphenyl) oder durch —SH, $—NH_2$, —COOH, —COOR°, $—CONH_2$, —CN oder Halogen substituiert sein kann. $Z_1$ und $Z_2$ können weiterhin $C_2—C_{10}$-Hydroxyalkyl (das durch einer oder mehrere NR°-Gruppen oder Q-Atome unterbrochen sein kann), $C_2—C_{18}$-Alkenyl, $C_3—C_{12}$-Cycloalkyl (das durch $C_1—C_4$-Alkyl, —OH, —SH, —COOR°, $—CONH_2$, —CN oder Halogen substituiert sein kann), $C_7—C_9$-Phenylalkyl, $C_7$-$C_{18}$-Alkylphenyl oder $C_6—C_{10}$-Aryl (das durch $C_1—C_{12}$-Alkoxy, $C_1—C_{12}$-Alkylthio, —COOH, —OH, $—NO_2$, oder Halogen substituiert sein kann) sein. $Z_1$ und $Z_2$ können auch gemeinsam eine gegebenenfalls verzweigte $C_3—C_7$-Alkylengruppe bilden, die durch O, S und NR° unterbrochen sein kann. Alle Amide enthalten mindestens eine Gruppe $—CONZ_1Z_2$. Partielle Amide (Halbamide) enthalten ausserdem noch mindestens eine Gruppe —COOH. Enthält das Amid mehrere Gruppen $—CONZ_1Z_2$, so können diese identisch oder verschieden sein.

Unter Imiden aliphatischer oder cycloaliphatischer Monocarbonsäuren sind hier symmetrische oder unsymmetrische Imide gemeint, die die Gruppe $—CO—N(Z_1)—CO—$ enthalten. Unter Imiden (cyclo-)-aliphatischer Di, Tri- oder Tetracarbonsäuren sind cyclische Imide gemeint, die eine Gruppe der Formel III oder IIIa enthalten:

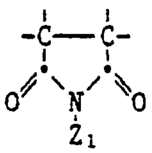

III

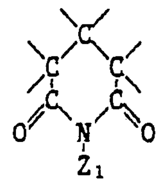

IIIa

Die erfindungsgemäss verwendbaren Korrosionsinhibitoren stellen Derivate des Benzthiazols dar. Besonders bevorzugt sind Halbamid-Derivate des Benzthiazols.

R als Alkyl, Alkoxy, Alkylthio oder Alkylsulfonyl enthält vorzugsweise 1—12 C-Atome, insbesondere 1—6 C-Atome. Beispiele hierfür sind Methyl, Ethyl, n-Propyl, Isopropyl, n-Butyl, Isobutyl, tert.-Butyl, n-Pentyl, n-Hexyl, n-Octyl, 1,1,3,3-Tetramethylbutyl, n-Nonyl, n-Decyl, n-Undecyl oder tert.-Dodecyl und die entsprechenden Alkoxy-, Alkylthio- und Alkylsulfonyl-Reste.

R als Cycloalkyl enthält bevorzugt 3—12 C-Atome, insbesondere 5—8 C-Atome, wie z.B. Cyclopropyl, Cyclobutyl, Cyclopentyl, Cyclohexyl, Cyclooctyl oder Cyclododecyl.

R als Halogenalkyl enthält vorzugsweise 1—4 C-Atome und 1—3 Fluor- oder Chloratome, wie z.B. Chlormethyl, Fluormethyl, Di- und Tri-fluormethyl oder 2-Chlorethyl.

R als Alkylphenyl enthält vorzugsweise 7—18 C-Atome und kann z.B. Tolyl, Xylyl, 4-Isopropylphenyl, 4-tert.Butylphenyl, 4-Octylphenyl oder 4-Dodecylphenyl sein. R als Phenylalkyl enthält vorzugsweise 7—9 C-Atome und kann z.B. Benzyl, 1-Phenylethyl, 2-Phenylethyl, α,α-Dimethylbenzyl oder 3-Phenylpropyl sein.

R als Halogen ist vorzugsweise Fluor, Chlor oder Brom. Wenn R —COOAlkyl ist, so enthält die Alkylgruppe vorzugsweise 1—4 C-Atome.

R als Amino- oder Carbamoyl-Gruppe hat vorzugsweise bis zu 20 C-Atome. Beispiele hierfür sind die Gruppen $—N(CH_3)_2$, $—N(C_2H_5)_2$, $—N(C_4H_9)_2$, $—N(Cyclohexyl)_2$, $—N(CH_3)$(Benzyl), Morpholino, Piperidino, $—CONH_2$, —CONHPhenyl, $—CONHC_8H_{17}$, $—CON(C_2H_5)_2$, $—CON(CH_2CH_2OH)_2$, Morpholinocarbonyl oder Piperidinocarbonyl.

Vorzugsweise ist einer der Substituenten R Wasserstoff, $C_1—C_4$-Alkyl oder $C_1—C_4$-Alkoxy und die anderen drei R sind Wasserstoff. Besonders bevorzugt sind alle vier Gruppen R Wasserstoff.

R°, $Z_1$ oder $Z_2$ als $C_1—C_{18}$-Alkyl kann gerade- oder verzweigtkettiges Alkyl sein, wie z.B. Methyl, Ethyl, n-Propyl, Isopropyl, n-Butyl, sec.-Butyl, tert.-Butyl, n-Pentyl, n-Hexyl, n-Heptyl, n-Octyl, 2-Ethylhexyl, tert-Octyl, n-Nonyl, n-Decyl, n-Dodecyl, n-Tetradecyl, n-Hexadecyl oder n-Octadecyl.

$Z_1$ oder $Z_2$ als durch O, S oder NR° unterbrochenes Alkyl kann z.B. 2-Methoxyethyl, 3-Ethoxypropyl, 2-Dodecyloxyethyl, 3,6-Dioxadecyl, 2-Butylthioethyl, 2-tert-Dodecylthioethyl, 2-(Dimethylamino-)propyl, 2-(Dibutylamino)ethyl, 3-Butylaminopropyl oder 2-(Methyl-phenylamino)-ethyl sein.

$Z_1$ oder $Z_2$ als durch SH, $NH_2$, COOH, COOR°, $CONH_2$, CN oder Halogen substituiertes Alkyl kann z.B. 2-Mercaptobutyl, 3-Mercaptopropyl, 2-Aminoethyl, 6-Aminohexyl, 5-Carboxypentyl, 2-Carboxyethyl, Ethoxycarbonylmethyl, 2-Isopropoxycarbonylethyl, 2-Carbamoylethyl, 2-Cyanoethyl, 2-Chlorethyl oder 3-Brompropyl sein.

$Z_1$ oder $Z_2$ als gegebenenfalls durch O oder NR° unterbrochenes $C_2—C_{10}$-Hydroxyalkyl kann z.B. 2-Hydroxyethyl, 2-Hydroxypropyl, 4-Hydroxybutyl, 6-Hydroxyhexyl, 5-Hydroxy-3-oxapentyl, 8-Hydroxy-3,6-dioxaoctyl, 11-Hydroxy-3,6,9-trioxaundecyl, 5-Hydroxy-3-(methylaza)-pentyl oder 9-Hydroxy-3,7-di(methylaza)-nonyl sein.

Wenn $Z_1$ oder $Z_2$ $C_2—C_{18}$ ist, ist es insbesondere Alkenylmethyl, wie z.B. Allyl, Methallyl, 2-Butenyl oder Oleyl. $Z_1$ oder $Z_2$ als gegebenenfalls substituiertes $C_3—C_{12}$-Cycloalkyl kann z.B. Cyclopropyl, Cyclobutyl, Cyclopentyl, Cyclohexyl, Cyclooctyl, Cyclodecyl, Methylcyclohexyl, Dimethylcyclohexyl, tert-Butylcyclohexyl, 3-Hydroxycyclohexyl, 4-Mercaptocyclohexyl, 2-Ethoxycarbonylcyclohexyl, 4-Carbamoylcyclohexyl,

3,4-Dichlorcyclopentyl, 4-Fluorcyclohexyl oder 4-Cyanocyclohexyl sein. Vorzugsweise ist es eine $C_5$—$C_8$-Cycloalkylgruppe.

Wenn R°, $Z_1$ oder $Z_2$ $C_7$—$C_9$-Phenylalkyl est, kann es z.B. Benzyl, 1-Phenylethyl, 2-Phenylethyl, α,α-Dimethylbenzyl oder 3-Phenylpropyl sein. Wenn R°, $Z_1$ oder $Z_2$ $C_7$—$C_{18}$-Alkylphenyl ist, so kann es z.B. Tolyl, Xylyl, 4-Isopropylphenyl, 4-tert.Butylphenyl, 4-Octylphenyl oder 4-Dodecylphenyl sein.

$Z_1$ oder $Z_2$ als gegebenenfalls substituiertes Aryl kann, z.B. Phenyl, 3-Chlorphenyl, 2,4-Dichlorphenyl, 4-Nitrophenyl, 3-Hydroxyphenyl, 4-Methoxyphenyl, 3-Isopropoxyphenyl, 4-(Methylthio)phenyl, 2-Carboxyphenyl, 1-Naphthyl, 2-Naphthyl oder 4-Chlor-1-naphthyl sein.

Bevorzugt ist R° Wasserstoff oder $C_1$—$C_4$-Alkyl. $Z_1$ und $Z_2$ sind vorzugsweise, H, $C_1$—$C_{12}$-Alkyl, $C_2$—$C_4$-Hydroxyalkyl, $C_3$—$C_{12}$-Alkoxyalkyl, Cyclohexyl, Benzyl, Phenyl, Tolyl oder Naphthyl oder $Z_1$ und $Z_2$ sind zusammen Tetramethylen, Pentamethylen oder 3-Oxapentamethylen.

Bevorzugte Korrosionsinhibitoren sind solche der Formel II

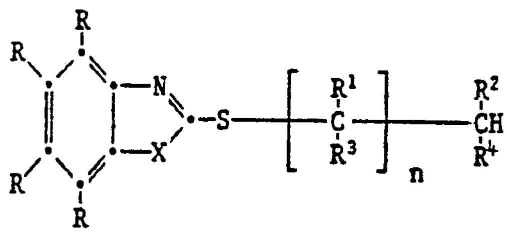

II

worin X und R die vorhin gegebene Bedeutung haben, n null oder 1 ist, und $R^1$, $R^2$, $R^3$ und $R^4$ unabhängig voneinander, Wasserstoff, Alkyl, Hydroxyalkyl, Halogenalkyl, Cyanoalkyl, Alkoxyalkyl, unsubstituiertes oder im Phenylkern durch Halogen, Nitro, Alkyl, Alkoxy, Carboxy oder Hydroxy substituiertes Phenyl oder Phenylalkyl, eine Gruppe —COOH, —CN oder —$CONZ_1Z_2$, worin $Z_1$ under $Z_2$ die vorhin gegebene Bedeutung haben oder durch 1, 2 oder 3 Gruppen —COOH, —CN oder —$CONZ_1Z_2$ substituiertes Alkyl bedeuten,

oder die entsprechenden Imide, die eine Gruppe der Formel III oder IIIa enthalten,

oder solche Verbindungen der Formel II, worin n = 1 ist und $R^1$ und $R^2$ zusammen, oder $R^1$ und $R^3$ zusammen eine gegebenenfalls verzweigte Alkylengruppe bilden, die durch eine oder zwei Gruppen —COOH oder —$CONZ_1Z_2$ substituiert sein kann,

oder solche Verbindungen der Formel II, worin n = 1 ist und $R^1$ und $R^2$ zusammen eine direkte Bindung bilden,

wobei der Rest IV

$$-\!\!-\!\!\left[C(R^1)(R^3)-\!\!-\right]_n CH(R^2)(R^4) \qquad \text{IV}$$

mindestens eine Gruppe —$CONZ_1Z_2$ oder —CN oder eine Gruppe der Formel III oder IIIa enthält,

sowie nicht-toxische Basenadditions-Salze solcher Verbindungen der Formel II, die eine Carboxylgruppe enthalten.

$R^1$, $R^2$, $R^3$ und $R^4$ als Alkyl sind vorzugsweise $C_1$—$C_{18}$-Alkyl, wie z.B. Methyl, Ethyl, n-Propyl, Isopropyl, n-Butyl, Isobutyl, tert.-Butyl, Pentyl, Hexyl, Octyl, Dodecyl oder Octadecyl. Als Hydroxyalkyl, Halogenalkyl oder Cyanoalkyl haben diese Substituenten vorzugsweise 1—4 C-Atome. Beispiele hierfür sind Hydroxymethyl, 1- oder 2-Hydroxyethyl, 2- oder 3-Hydroxypropyl, Chlormethyl, Bromethyl, Bromisopropyl, Cyanomethyl oder 2-Cyanoethyl. Als Alkoxyalkyl haben diese Substituenten vorzugsweise 2—10 C-Atome. Beispiele hierfür sind Methoxymethyl, 1-Methoxymethyl, 2-Ethoxypropyl, 1-Methoxybutyl, n-Butoxymethyl oder 4-Isopropoxybutyl.

$R^1$, $R^2$, $R^3$ und $R^4$ als durch —COOH oder $CONZ_1Z_2$ substituiertes Alkyl hat vorzugsweise 2—12 C-Atome. Beispiele hierfür sind die Gruppen

—$CH_2CONHC_4H_9$, —$CH_2CONHC_8H_{17}$, —$CH_2CON(CH_3)_2$, —$CH_2CH_2CONH_2$, —$CH_2CH_2CONHCH_3$,

—$CH_2CH_2CONHC_{12}H_{25}$, —$(CH_2)_3CONHC_6H_{13}$, —$(CH_2)_3$CONHCyclohexyl, —$CH_2$CONHPhenyl,

—CH(COOH)—$CH_2$—$CONHC_8H_{17}$, CH($CONHC_4H_9$)—$CH_2$—$CONHC_4H_9$, —CH(COOH)—$CH_2CON(C_2H_5)_2$,

—CH(COOH)—CH(CONHBenzyl)—$CH_2$COOH oder

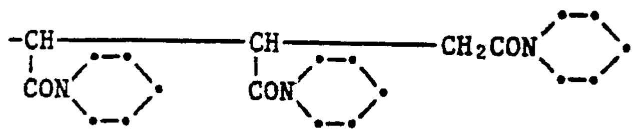

Dieselben Substituenten als unsubstituiertes oder substituiertes Phenyl oder Phenylalkyl können z.B. 4-Chlorphenyl, 3-Nitrophenyl, Tolyl, Xylyl, 3-Methoxyphenyl, 4-Isopropylphenyl, 3-Carboxyethyl, 4-Hydroxyphenyl, 4-Brombenzyl, 4-tert.-Butylbenzyl, 2-Phenylethyl oder 3-Phenylpropyl, vorzugsweise jedoch Phenyl oder Benzyl sein. Wenn $R^1$ und $R^2$ oder $R^1$ und $R^3$ zusammen Alkylen sind, so bilden sie zusammen mit den C-Atomen, an die sie gebunden sind, einen Cycloalkanring, vorzugsweise einen Cyclopentan oder Cyclohexanring, der durch Alkylgruppen, vorzugsweise $C_1$—$C_4$-Alkylgruppen, oder durch 1 oder 2 Gruppen, —CN, —COOH oder —$COZ_1Z_2$ substituiert sein kann.

Wenn $R^1$ und $R^2$ zusammen eine direkte Bindung bedeuten, so stellen die Verbindungen der Formel II Amide, Imide oder Nitrile ungesättigter Carbonsäuren dar.

Bevorzugt sind $R^1$, $R^2$, $R^3$ und $R^4$ Wasserstoff, $C_1$—$C_4$-Alkyl, —CN, —COOH, —$CONZ_1Z_2$ oder durch —CN, —COOH oder —$CONZ_1Z_2$ substituiertes Alkyl. Besonders bevorzugt sind Verbindungen der Formel II, worin $R^4$ einer Gruppe —COOH, —$CONZ_1Z_2$ oder durch —COOH oder —$CONZ_1Z_2$ substituiertes Alkyl ist. Bevorzugt sind weiterhin Verbindungen der Formel II, worin n = 1 ist und mindestens zwei der Reste $R^1$, $R^2$, $R^3$ und $R^4$ eine Gruppe —COOH, —$CONZ_1Z_2$ oder durch —COOH oder —$CONZ_1Z_2$ substituiertes Alkyl sind, insbesondere solche Verbindungen der Formel II, die entweder zwei Gruppen —$CONZ_1Z_2$ oder eine Gruppe —COOH und eine Gruppe —$CONZ_1Z_2$ an benachbarten C-Atomen gebunden enthalten.

Verbindungen der Formel II, die eine Carboxylgruppe enthalten, können Basen-Additionssalze bilden. Nicht-toxische Basen-Additionssalze sind Metall-, Ammonium- und organische Ammoniumsalze, insbesondere Salze von Alkalimetallen, Erdalkalimetallen, von Metallen der Gruppen IIB, IIIA oder VIII des periodischen Systems der Elemente, Ammoniumsalze oder Salze von organischen Aminen. Beispiele hierfür sind insbesondere Natrium-, Kalium-, Calcium-, Magnesium-, Zink-, Aluminium-, Ammonium-, Trialkylammonium- und Tris(hydroxyethyl)ammonium-Salze.

Spezifische Beispiele der Verbindungen der Formel I umfassen:

N-Methyl-benzthiazol-2-ylthioacetamid
N-Phenyl-benzthiazol-2-ylthioacetamid
N-Ethyl-(5-trifluoromethylbenzthiazol-2-ylthio)acetamid
N-n-Propyl-(5-carboxybenzthiazol-2-ylthio)acetamid
N-n-Octyl-(5-ethoxycarbonylbenzthiazol-2-ylthio)acetamid
N-n-Dodecyl-(6-methylsulphonylbenzthiazol-2-ylthio)acetamid
N-Ethyl-3-(benzthiazol-2-ylthio)propionamid
N-Allyl-3-(benzthiazol-2-ylthio)propionamid
N-Cyclohexyl-3-(benzthiazol-2-ylthio)propionamid
N-n-Octadecyl-3-(benzthiazol-2-ylthio)propionamid
N-Methyl-3-(6-aminobenzthiazol-2-ylthio)propionamid
N-Cyclohexyl-3-(6-aminobenzthiazol-2-ylthio)propionamid
N-Ethyl-3-(benzthiazol-2-ylthio)-2-methylpropionamid
N-iso-Butyl-4-(benzthiazol-2-ylthio)butyramid
N-Ethyl-3-(benzthiazol-2-ylthio)butyramid
N-2-Ethylhexyl-3-(benzthiazol-2-ylthio)butyramid
N-2-Methoxyethyl-3-(benzthiazol-2-ylthio)-3-methylbutyramid
N,N'-Diisopropyl-benzthiazol-2-ylthiomalonsäurediamid
N,N'-Dimethyl-benzthiazol-2-ylthiobernsteinsäurediamid
N,N'-Diethyl-benzthiazol-2-ylthiobernsteinsäurediamid
N,N'-Diisopropyl-benzthiazol-2-ylthiobernsteinsäurediamid
N,N'-Di-n-butyl-benzthiazol-2-ylthiobernsteinsäurediamid
N,N'-Di-i-octyl-benzthiazol-2-ylthiobernsteinsäurediamid
N,N'-Di-n-decyl-benzthiazol-2-ylthiobernsteinsäurediamid
N,N'-Di-n-octadecyl-benzthiazol-2-ylthiobernsteinsäurediamid
N,N'-Di-2-ethylhexyl-benzthiazol-2-ylthiobernsteinsäurediamid
N,N'-Dicyclohexyl-benzthiazol-2-ylthiobernsteinsäurediamid
N,N'-Diphenyl-benzthiazol-2-ylthiobernsteinsäurediamid
N,N'-Dibenzyl-benzthiazol-2-ylthiobernsteinsäurediamid
N,N'-Di-(4-methylphenyl)benzthiazol-2-ylthiobernsteinsäurediamid
N,N'-Butyl-methyl-benzthiazol-2-ylthiobernsteinsäurediamid
N,N'-Methyl-phenyl-benzthiazol-2-ylthiobernsteinsäurediamid
N,N'-Ethyl-benzyl-benzthiazol-2-ylthiobernsteinsäurediamid
1-(Benzthiazol-2-ylthio)-2-methylcarbamoyl-ethan-1-carbonsäure
1-(Benzthiazol-2-ylthio)-1-ethylcarbamoyl-ethan-2-carbonsäure
1-(Benzthiazol-2-ylthio)-1-ethylhexylcarbamoyl-ethan-2-carbonsäure

N,N'-Di-n-propyl-(5-methylbenzthiazol-2-ylthio)bernsteinsäurediamid
N,N'-Di-n-hexyl-(6-ethylbenzthiazol-2-ylthio)bernsteinsäurediamid
N,N'-Di-but-3-enyl-(4-isopropylbenzthiazol-2-ylthio)bernsteinsäurediamid
N-Methyl-N'-allyl-(7-t-butylbenzthiazol-2-ylthio)bernsteinsäurediamid
N,N'-Diphenyl-(5-n-hexylbenzthiazol-2-ylthio)bernsteinsäurediamid
N,N'-Dicyclopentyl-(6-[1,1,3,3-tetramethylbutyl]-benzthiazol-2-ylthio)bernsteinsäurediamid
N,N'-Diphenyl-(6-cyclohexylbenzthiazol-2-ylthio)bernsteinsäurediamid
N,N'-Di-naphthyl-(7-benzylbenzthiazol-2-ylthio)bernsteinsäurediamid
N,N'-Dibenzyl-(6-methoxybenzthiazol-2-ylthio)bernsteinsäurediamid
N-Ethyl-N'-methyl-(5-methoxybenzthiazol-2-ylthio)bernsteinsäurediamid
N-Benzyl-N'-phenyl(5-ethoxycarbonylbenzthiazol-2-ylthio)bernsteinsäurediamid
1-(4-Methylbenzthiazol-2-ylthio)-2-methylcarbamoyl-ethan-1-carbonsäure
1-(6-Methylbenzthiazol-2-ylthio)-2-butylcarbamoyl-ethan-2-carbonsäure
N,N'-Di-1,1,3,3-tetramethylbutyl-(4-fluorbenzthiazol-2-ylthio)bernsteinsäurediamid
N,N'-Dioctadecyl-(7-brombenzthiazol-2-ylthio)bernsteinsäurediamid
N,N'-Di-n-nonyl-(6-chlorbenzthiazol-2-ylthio)bernsteinsäurediamid
N,N'-Dimethyl-(4-phenylbenzthiazol-2-ylthio)bernsteinsäurediamid
N,N'-Diethyl-(6-nitrobenzthiazol-2-ylthio)bernsteinsäurediamid
N,N'-Diisopropyl-(5-cyanobenzthiazol-2-ylthio)bernsteinsäurediamid
N,N'-Di-iso-butyl-(5-carboxybenzthiazol-2-ylthio)bernsteinsäurediamid
N,N'-Di-n-hexyl-(7-hydroxybenzthiazol-2-ylthio)bernsteinsäurediamid
N,N'-Di-ethoxyethyl-(6-chloro-4-methylbenzthiazol-2-ylthio)bernsteinsäurediamid
1-(5-Chlor-6-n-butylbenzthiazol-2-ylthio)-2-n-octylcarbamoyl-ethan-2-carbonsäure
1-(4-Bromo-5-benzylbenzthiazol-2-ylthio)-2-benzylcarbamoyl-ethan-2-carbonsäure
1-(5-Nitro-6-n-propylbenzthiazol-2-ylthio)-2-phenylcarbamoyl-ethan-2-carbonsäure
1-(5-Brom-6-n-propoxybenzthiazol-2-ylthio)-2-naphthylcarbamoyl-ethane-2-carbonsäure
1-(6-Amino-benzthiazol-2-ylthio)-2-allylcarbamoyl-ethan-2-carbonsäure
1-(6-Methylaminobenzthiazol-2-ylthio)-2-cyclohexylcarbamoyl-ethan-2-carbonsäure
1-(5-Dimethylaminobenzthiazol-2-ylthio)-2-methoxyethylcarbamoyl-ethan-2-carbonsäure
1-(7-Phenylaminobenzthiazol-2-ylthio)-2-n-octylcarbamoyl-ethan-2-carbonsäure
1-(6-Diphenylaminobenzthiazol-2-ylthio)-2-n-decylcarbamoyl-ethan-2-carbonsäure
1-(4-Benzylaminobenzthiazol-2-ylthio)-2-n-octadecylcarbamoyl-ethan-2-carbonsäure
N,N'-Dimethyl-(4-morpholinobenzthiazol-2-ylthio)bernsteinsäurediamid
N,N'-Diethyl-(5-carbamoylbenzthiazol-2-ylthio)bernsteinsäurediamid
N,N'-Di-n-propyl-(5-methylcarbamoylbenzthiazol-2-ylthio)bernsteinsäurediamid
N,N'-Di-n-butyl-(5-diethylcarbamoylbenzthiazol-2-ylthio)bernsteinsäurediamid
N,N'-Di-1,1,3,3-tetramethylbutyl-(6-phenylcarbamoylbenzthiazol-2-ylthio)bernsteinsäurediamid
N,N'-Diphenyl-(5,6-dimethyl-benzthiazol-2-ylthio)bernsteinsäurediamid
N,N'-Dibenzyl-(4,5,6-triethylbenzthiazol-2-ylthio)bernsteinsäurediamid
N,N'-Diethyl-(4,5,6,7-tetramethylbenzthiazol-2-ylthio)bernsteinsäurediamid
N,N'-Diethyl-(benzthiazol-2-ylthio)-propan-1,2-dicarbonsäurediamid
N,N'-Diethyl-3-(benzthiazol-2-ylthio)-propan-1,2-dicarbonsäurediamid
1-(Benzthiazol-2-ylthio)-3-ethylcarbamoyl-propan-2-carbonsäure
N,N'-Dimethyl-3-(6-trifluoromethylbenzothiazol-2-ylthio)-propan-1,2-dicarbonsäurediamid
N,N'-Di-n-butyl-3-(6-carbmethoxybenzthiazol-2-ylthio)-propan-1,2-dicarbonsäurediamid
N,N'-Di-n-octyl-3-(6-aminobenzthiazol-2-ylthio)-propan-1,2-dicarbonsäurediamid
N,N'-Dibenzyl-3-(5-ethylaminobenzthiazol-2-ylthio)-propan-1,2-dicarbonsäurediamid
N-Methyl-N'-octadecyl-3-(4-dibutylaminobenzthiazol-2-ylthio)-propan-1,2-dicarbonsäurediamid
N,N'-Diphenyl-4-(morpholinobenzthiazol-2-ylthio)-propan-1,2-dicarbonsäurediamid
N,N'-Dimethyl-(1-benzthiazol-2-ylthio)-propan-1,3-dicarbonsäurediamid
N,N'-Diisopropyl-2-(benzthiazol-2-ylthio)-propan-1,3-dicarbonsäurediamid
N,N'-Dimethyl-3-(benzthiazol-2-ylthio)-phenylpropan-1,2-dicarbonsäurediamid
N,N'-Diethyl-3-(benzthiazol-2-ylthio)-3-(4-carboxyphenyl)-propan-1,2-dicarbonsäurediamid
N,N-Di-n-butyl-3-(benzthiazol-2-ylthio)-3-(2,4-dicarboxyphenyl)-propane-1,2-dicarbonsäurediamid
N,N'-Di-n-hexyl-3-(benzthiazol-2-ylthio)-3,3-diphenylpropane-1,2-dicarbonsäurediamid
N,N'-Dimethyl-1-(benzthiazol-2-ylthio)-butane-1,2-dicarbonsäurediamid
N,N'-Dibenzyl-1-(benzthiazol-2-ylthio)-2-methylpropan-1,2-dicarbonsäurediamid
N,N'-Di-allyl-2-(benzthiazol-2-ylthio)-butan-2,3-dicarbonsäurediamid
N,N'-Diphenyl-1-(benzthiazol-2-ylthio)-butan-2,4-dicarbonsäurediamid
N,N',N''-Trimethyl-4-(benzthiazol-2-ylthio)-butan-1,2,3-dicarbonsäurediamid
1-(Benzthiazol-2-ylthio)-3,4-bis(methylcarbamoyl)-butan-2-carbonsäure
N,N'-Diethyl-1-(benzthiazol-2-ylthio)-pentan-1,5-dicarbonsäurediamid
N,N''-Di-n-hexyl-3-(benzthiazol-2-ylthio)-hexan-1,2-dicarbonsäurediamid
N,N',N'',N'''-Tetraethyl-8-(benzthiazol-2-ylthio)-octan-1,3,5,7-tetracarbonsäuretetramid
N,N'-Dimethyl-1-(benzthiazol-2-ylthio)-cyclohexan-1,2-dicarbonsäurediamid

N,N'-Diphenyl-4-(benzthiazol-2-ylthio)-cyclohexan-1,2-dicarbonsäurediamid
N,N',N''-Tri-n-octyl-1-(benzthiazol-2-ylthio)-propan-1,2,3-tricarbonsäuretriamid
N,N'-Di-n-pentyl-1-(benzthiazol-2-ylthio)-3-chlorpropan-1,2-dicarbonsäurediamid
N,N'-Di-n-nonyl-1-(benzthiazol-2-ylthio)-3-methoxypropane-1,2-dicarbonsäurediamid
N,N'-Di-n-decyl-1-(benzthiazol-2-ylthio)-3-methoxypropane-1,2-dicarbonsäurediamid
N,N'-Dimethyl-1-(benzthiazol-2-ylthio)-2-phenylbernsteinsäurediamid
N,N'-Diethyl-1-(benzthiazol-2-ylthio)-2-benzylbernsteinsäurediamid
N,N-Diethyl-2,3-bis-(benzthiazol-2-ylthio)-butan-1,4-dicarbonsäurediamid
Natrium-1-(benzthiazol-2-ylthio)-2-methylcarbamoyl-ethan-1-carboxylat
Kalium-1-(benzthiazol-2-ylthio)-2-ethylcarbamoyl-ethan-2-carboxylat
Calcium-1-(benzthiazol-2-ylthio)-2-ethylcarbamoyl-ethan-2-carboxylat
Zink-1-(benzthiazol-2-ylthio)-2-isopropylethan-2-carboxylat
Cobalt-1-(benzthiazol-2-ylthio)-2-n-butylcarbamoyl-ethan-2-carboxylat
Aluminium-1-(benzthiazol-2-ylthio)-2-ethoxyethylcarbamoyl-ethan-2-carboxylat
Ammonium-1-(benzthiazol-2-ylthio)-2-n-octylcarbamoyl-ethan-2-carboxylat
Methylammonium-1-(benzthiazol-2-ylthio)-2-methylcarbamoyl-ethan-2-carboxylat
Triethanolammonium-1-(benzthiazol-2-ylthio)-2-methylcarbamoyl-ethan-2-carboxylat
Octylammonium-1-(benzthiazol-2-ylthio)-2-phenylcarbamoyl-ethan-2-carboxylat
Cyclohexylammonium-1-(benzthiazol-2-ylthio)-2-benzylcarbamoyl-ethan-2-carboxylat
Diethylammonium-1-(benzthiazol-2-ylthio)-2-n-butylcarbamoyl-ethan-2-carboxylat
Tributylammonium-1-(benzthiazol-2-ylthio)-2-methylcarbamoyl-ethan-2-carboxylat
Natrium-3-(benzthiazol-2-ylthio)-2-ethylcarbamoyl-propan-1,2-dicarboxylat
Kalium-3-(benzthiazol-2-ylthio)-2-n-propylcarbamoyl-propan-1,2-dicarboxylat
Calcium-3-(benzthiazol-2-ylthio)-2-methylcarbamoyl-propan-1,2-dicarboxylat
Zink-3-(benzthiazol-2-ylthio)-2-benzylcarbamoyl-propan-1,2-dicarboxylat
Aluminium-3-(benzthiazol-2-ylthio)-2-phenylcarbamoyl-propan-1,2-dicarboxylat
Ammonium-3-(benzthiazol-2-ylthio)-2-allylcarbamoyl-propan-1,2-dicarboxylat
N,N'-Diisopropyl-1-(benzthiazol-2-ylthio)-propene-1,2-dicarbonsäurediamid
N,N'-Di-n-butyl-2-(benzthiazol-2-ylthio)-but-1-ene-2,3-dicarbonsäurediamid
Benzthiazol-2-ylthiosuccinimid
5-Methylbenzthiazol-2-ylthiosuccinimid
6-Aminobenzthiazol-2-ylthiosuccinimid
5,6-Dimethylbenzthiazol-2-ylthiosuccinimid
4,5,6-Triethylbenzthiazol-2-ylthiosuccinimid
4,5,6,7-Tetramethylbenzthiazol-2-ylthiosuccinimid
3-(Benzthiazol-2-ylthio)-propan-1,2-dicarbonsäureimid
4-(Benzthiazol-2-ylthio)-3-ethoxycarbonyl-butan-1,2-dicarbonsäureimid
N-Methyl-benzthiazol-2-ylthiosuccinimid
N-n-Octyl-benzthiazol-2-ylthiosuccinimid
N-Phenyl-benzothiazol-2-ylthiosuccinimid
N-Methyl-3-(benzothiazol-2-ylthio)-propan-1,2-dicarbonsäureimid
N-n-Dodecyl-3-(benzothiazol-2-ylthio)-propan-1,2-dicarbonsäureimid
Benzothiazol-2-ylthio-acetonitril
2-(Benzothiazol-2-ylthio)-propionitril
Benzothiazol-2-ylthio-bernsteinsäuredinitril
Benzothiazol-2-ylthio-malondinitril
Benzothiazol-2-ylthiomethyl-bernsteinsäuredinitril
N,N,N',N'-Tetraethyl-benzthiazol-2-ylthiobernsteinsäurediamid
N,N,N',N'-Tetra-n-octyl-benzthiazol-2-ylthiobernsteinsäurediamid
N-Hydroxyethyl benzthiazol-2-ylthioacetamid
N-Hydroxyethyl 3-(benzthiazol-2-ylthio)propionamid
N,N'-Bis(hydroxyethyl)-benzthiazol-2-ylthiobernsteinsäurediamid
N,N'-Bis(morpholino)-benzthiazol-2-ylthiobernsteinsäurediamid
1-(Benzothiazol-2-ylthio)-2-(hydroxyethylcarbamoyl)ethan-carbonsäure
1-(Benzothiazol-2-ylthio)-3-(hydroxyethylcarbamoyl)propan-2-carbonsäure
1-(Benzothiazol-2-ythio)-2-(diethylcarbamoyl)ethan-1-carbonsäure
1-(Benzothiazol-2-ythio)-3-(dicyclohexylcarbamoyl)propan-2-carbonsäure.

Einige der erfindungsgemässen Korrosionsinhibitoren sind bekannte Verbindungen. So sind in Chem. Abstracts *69* (1968), 96541, Verbindungen der Formel

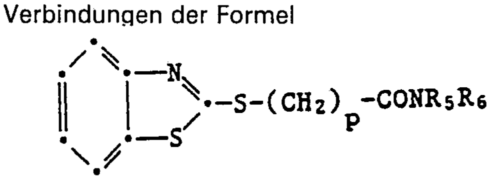

beschreiben, worin p 1 oder 2 ist und $R_5$ und $R_6$ Wasserstoff, Methyl, Phenyl, p-Tolyl, o-Methoxyphenyl, m-Nitrophenyl oder Benzyl sind oder $-NR_5R_6$ Morpholino oder Piperidino ist.

β-(Benzthiazol-2-ylthio)propionsäureamid ist im GB—Patent 564,412 beschreiben, β-(Benzthiazol-2-ylthio)buttersäureamid ist in der GB—A—2,019,392 beschreiben, N-Hydroxyethyl-(benzthiazol-2-ylthio)acetamid ist in Rev. Roum. Chim. *28* (1983), 757 beschreiben und N-Phenyl-benzthiazol-2-ylthioacetamid im GB—Patent 448.414.

Verbindungen der Formel

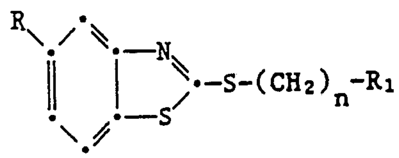

worin R Wasserstoff oder Chlor ist, n 1—3 ist und $R_1$ eine Amid- oder Nitrilgruppe ist, sind in der EP—A—6347 als Pflanzenwuchsregulatoren beschrieben.

N-Ethyl-(benzthiazol-2-ylthio)-succinimid und N-Ethyl-(benzoxazol-2-ylthio)-succinimid sind beschrieben in der DE—OS 2,250,136.

Ein grosser Teil der erfindungsgemäss verwendbaren Korrosionsinhibitoren sind jedoch neue Verbindungen und als solche ebenfalls Gegenstand der Erfindung.

Eine bevorzugt Gruppe neuer Verbindungen sind Verbindungen der Formel II, die 2, 3 oder 4 Amidgruppen $-CONZ_1Z_2$ enthalten. Eine weitere bevorzugte Gruppe neuer Verbindungen sind die partiellen Amide der Formel II, die im Rest

$$-\!\left[C(R^1)(R^3)-\right]_n CH(R^2)(R^4)$$

mindestens eine Gruppe $-CONZ_1Z_2$ und mindestens eine Gruppe —COOH enthalten.

Eine weitere bevorzugte Gruppe neuer Verbindungen sind die Imide der Formel II, worin n = 1 ist und $R^1$ und $R^3$ Wasserstoff sind und die eine oder zwei Gruppen der Formel III oder IIIa enthalten.

Eine weitere bevorzugte Gruppe neuer Verbindungen sind die Nitrile der Formel II, die 2, 3 oder 4 Cyanogruppen enthalten.

Die bekannten wie die neuen Verbindungen können nach verschiedenen Verfahren hergestellt werden.

Ein bevorzugtes Herstellungsverfahren ist die Addition eines heterocyclischen Mercaptanes der Formel V

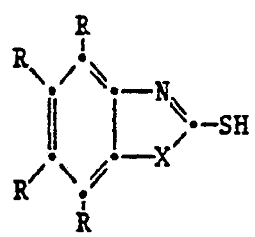

V

an ein α,β-ungesättigtes Amid, Imid oder Nitril. Diese Reaktion wird bevorzugt in stark saurem Medium durchgeführt und ist Gegenstand einer separaten Patentanmeldung.

Ein anderes, allgemein verwendbares Herstellungsverfahren für Verbindungen der Formel II ist die Umsetzung von Verbindungen der Formel VI mit Verbindungen der Formel VII

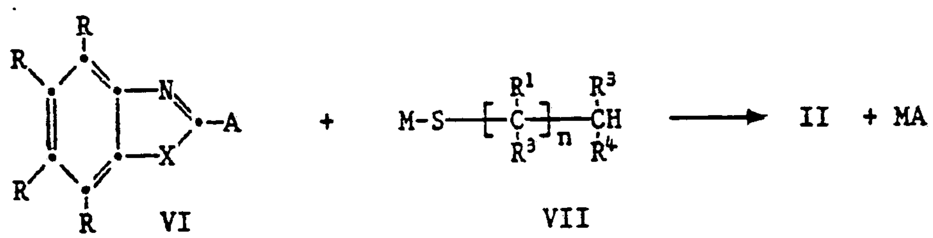


VI VII

Hierin bedeutet A eine Abgangsgruppe, wie z.B. Cl, Br oder Tosyloxy, M bedeutet H oder ein Kation, wie z.B. ein Alkali-, Erdalkali- oder Ammonium-Kation, und R, X, $R^1$, $R^2$, $R^3$, $R^4$ haben die eingangs gegebene Bedeutung.

Eine Variante dieses Verfahrens ist die Umsetzung einer Verbindung der Formel VIII mit einer Verbindung der Formel IX

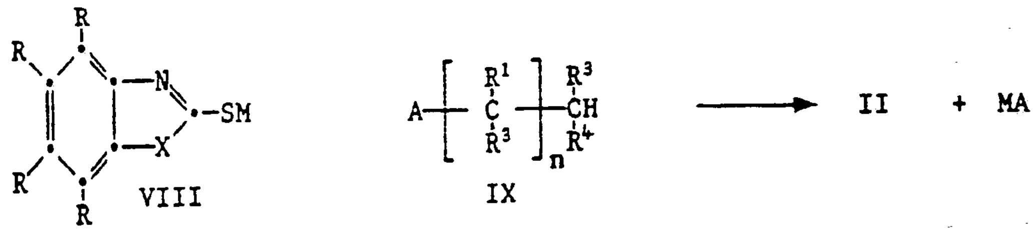

VIII IX II + MA

Beide Reaktionen werden vorzugsweise in Gegenwart von Basen ausgeführt.

Die erfindungsgemäss verwendbaren Amide können weiterhin aus den entsprechenden Carbonsäuren oder deren Estern oder Chloriden durch Umsetzung mit einem Amin $Z_1$—NH—$Z_2$ hergestellt werden nach den allgemein bekannten Methoden der N-Acylierung

Partielle Amide (Halbamide) lassen sich vorzugsweise aus den entsprechenden cyclischen Dicarbonsäureanhydriden durch Umsetzung mit einem Aequivalent $Z_1$—NH—$Z_2$ herstellen. Setzt man solche Dicarbonsäureanhydride mit einem primären Amin $Z_1$—$NH_2$ um, so erhält man zunächst ein Halbamid, das sich bei höherer Temperatur in ein cyclisches Imid umwandeln lässt.

Die cyclischen Dicarbonsäureanhydride sind daher wichtige Zwischenprodukte für die Synthese der erfindungsgemäss verwendbaren Halbamide und Imide. Besonders gilt dies für Anhydride der Formel X und XI

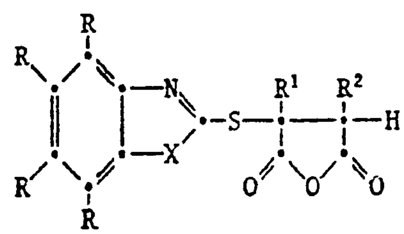

X

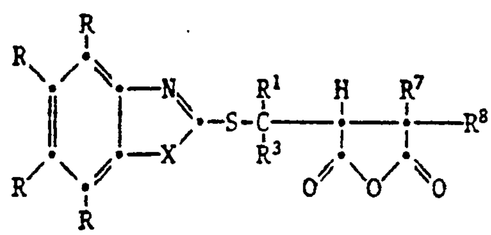

XI

worin $R^7$ und $R^8$ Wasserstoff oder $C_1$—$C_{10}$-Alkyl bedeutet, das durch —COOR oder —$CONZ_1Z_2$ substituiert sein kann, und die Summe der C-Atome in $R^7$ und $R^8$ nicht grösser als 10 ist. Vorzugsweise sind $R^7$ und $R^8$ Wasserstoff.

Solche Anhydride können durch Dehydratisierung der entsprechenden Dicarbonsäuren oder durch Addition ungesättigter Dicarbonsäureanhydride an Verbindungen der Formel V hergestellt werden.

Die erfindungsgemäss verwendbaren Nitrile können durch Dehydratisierung der entsprechenden primären Amide hergestellt werden. Bevorzugt ist die Herstellung durch Addition ungesättigter Nitrile an V im sauren Medium.

All diese Verbindungen mit der Gruppe der Formel I, insbesondere die Verbindungen der Formel II sind verwendbar als Korrosionsinhibitoren a) in Anstrichstoffen oder b) in wässrigen Systemen ausserhalb der Anstrichstoffe.

Die Anstrichstoffe enthalten als Hauptbestandteil ein Bindemittel das in organischer Lösung, in wässriger Dispersion oder Lösung oder in fester Form eingesetzt werden kann. Bindemittel für nichtwässrige Applikationen sind z.B. Epoxidharze (in Kombination mit einem Epoxid-Härter), Polyurethane, Aminoplaste, Acrylharze, Polyester oder Alkylharze und deren Mischungen. Weitere Beispiele für solche Bindemittel sind Phenolharze, Vinylharze wie Polyvinylchlorid, Polyvinylbutyrat oder Polyvinylacetat und deren Copolymere, Chlorkautschuk und andere chlorierte Harze, Styrol-Copolymerisate wie Styrol-Butadien, Celluloseester, Leinölharze und sonstige trocknende Oele.

Für wässrige Anstrichstoffe eigen sich wasserlösliche oder wasser-dispergierbare Bindemittel. Dies können beispielsweise Alkydharze, Polyester, Acrylharze, Polyurethane, Epoxidharze, Phenoplaste, Aminoplaste und deren Mischungen sein, weiterhin Mono- und Copolymere von Vinylethern, Vinylestern, Styrol, Vinylidenchlorid und Vinylchlorid. Die Härtung dieser wässrigen Anstrichmittel kann durch

Vernetzung der Bindemittelharze mit z.B. Aminoplasten, Phenoplasten, blockierten Isocyanaten, Epoxidharzen, aktivierten Carbonsäureestern oder Mannich-Basen von Phenolen bewirkt werden. Die für wässrige Anstrichstoffe verwendeten Bindemittel sind oft durch Einbau basischer oder saurer Gruppen modifizierte Harze. Durch Neutralisation durch Gruppen werden diese Harze wasserlöslich oder wasserdispergierbar.

Die Anstrichstoffe können ausser dem Bindemittel und dem erfindungsgemässen Korrosionsinhibitor weitere Komponenten enthalten, wie z.B. Pigmente, Farbstoffe, Extender oder andere Additive wie sie für Anstrichstoffe üblich sind. Das Pigment kann ein organisches, anorganisches oder metallisches Pigment sein, z.B. Titandioxid, Eisenoxid, Aluminiumbronze, Phthalocyaninblau etc. Man kann zusätzlich auch antikorrosive Pigmente verwenden, wie z.B. Phosphat- oder Borat-enthaltende Pigmente, Metallpigmente oder Metalloxidpigmente, wie sie in Farbe und Lack *88,* 183 (1982) genannt sind oder wie sie im EP—A1—54267 aufgeführt sind. Beispiele für verwendbare Extender sind Talkum, Kalk, Aluminiumoxid, Baryt, Glimmer oder Kieselerden. Beispiele für sonstige Zusatzstoffe sind Verlaufhilfsmittel, Dispersionsmittel, Thioxotropiemittel, Haftverbesserer, Antioxydantien, Lichtschutzmittel oder Härtungskatalysatoren.

Von besonderer Bedeutung ist der Zusatz basischer Füllstoffe oder Pigmente. Diese bewirken im bestimmten Bindemittelsystemen wie z.B. in Acryl- und Alkylharzen einen synergistischen Effekt auf die Korrosionsinhibierung. Beispiele für solche basische Füllstoffe oder Pigmente sind Calcium- oder Magnesiumcarbonat, Zinkoxid, Zinkcarbonat, Zinkphosphat, Magnesiumoxide, Aluminiumoxid, Aluminiumphosphat oder Gemische davon. Beispiele für Pigmente sind z.B. solche auf Basis von Aminoanthrachinon.

Die erfindungsgemässen Korrosionsinhibitoren können auch zuerst auf solche basische Füllstoffe oder Pigmente aufgebracht werden, beispielsweise durch Chemisorption aus wässriger Lösung, und die so erhaltenen Zubereitungen dem Ueberzugsmittel zugesetzt werden.

In einer weiteren bevorzugten Ausführungsform der Erfindung werden die Korrosionsinhibitoren zusammen mit basischen Ionenaustauschern verwenden oder man behandelt einen solchen Ionenaustauscher zuerst mit einer Lösung des Inhibitors und setzt diese Zubereitung dem Ueberzugsmittel, zu. Beispiele für basische Ionenaustauscher sind alle typischen Anionenaustauscher, wie z.B. unter den Namen Dowex® 1 oder 11 oder Amberlite®IRA im Handel sind.

Schliesslich kann der Korrosionsinhibitor auch auf einem neutralen Trägerstoff aufgebracht werden. Als Trägerstoffe eignen sich insbesondere pulverförmige Füllstoffe oder Pigmente. Diese Technik ist in der DE—OS 31 22 907 näher beschrieben.

Das Ueberzugsmittel kann ausser dem erfindungsgemässen Korrosionsinhibitor noch andere organische, metallorganische oder anorganische Korrosionsinhibitoren enthalten wie z.B. Salze der Nitroisophthalsäure, Tannin, Phosphorester, technische Amine, substituierte Benztriazole oder substituierte Phenole, wie sie z.B. in der DE—OS 31 46 265 beschrieben sind.

Die Korrosionsinhibitoren können dem Anstrichstoff während dessen Herstellung zugesetzt werden, z.B. während der Pigmentverteilung durch Mahlen oder man löst die Inhibitoren in einem Lösungsmittel vor und rührt die Lösung in den Anstichstoff ein. Man verwendet den Inhibitor in einer Menge von 0,1—20 Gew.-% vorzugsweise 0,5—5 Gew.-%, bezogen auf den Feststoffgehalt des Anstrichstoffes.

Die Erfindung betrifft daher auch antikorrosive Anstrichstoffe, die als Korrosionsinhibitor 0,1—20 Gew.-%, vorzugsweise 0,5—5 Gew.-% (bezogen auf den Feststoffgehalt) eines Amides, Imides oder Nitrils einer aliphatischen oder cycloaliphatischen Mono-, Di-, Tri- oder Tetracarbonsäure enthalten, welche im aliphatischen oder cycloaliphatischen Rest durch eine oder mehrere, vorzugsweise durch eine Gruppe der Formel I substituiert ist,

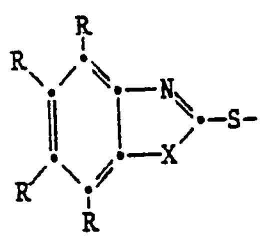

I

worin X Sauerstoff, Schwefel oder NH bedeutet und jedes R unabhängig voneinander Wasserstoff, Alkyl, Halogenalkyl, Alkoxy, Alkylthio, Alkylsulfonyl, Cycloalkyl, Phenyl, Alkylphenyl, Phenylalkyl, Halogen, —CN, —$NO_2$, —COOH, —COOAlkyl oder eine tertiäre Amino- oder Carbamoylgruppe bedeutet, sowie nichttoxische Salze solcher Verbindungen, die Carboxylgruppen enthalten.

Die erfindungsgemässen Anstrichstoffe werden vorzugsweise als Grundierung (Primer) auf metallischen Substraten, insbesondere auf Eisen, Stahl, Kupfer und Aluminium verwendet. Sie können dabei als sogenannte Konversions-Anstriche fungieren, indem an der Grenzfläche von Metall und Ueberzug chemische Reaktionen stattfinden. Der Auftrag der Ueberzüge kann nach den üblichen Methoden erfolgen wie Spritzen, Streichen, Aufrollen, Tauchen oder durch Elektroabscheidung, insbesondere kathodische Abscheidung. Je nachdem, ob der Filmbildner ein physikalisch trocknendes oder eine hitze-oder strahlenhärtbares Harz ist, erfolgt die Aushärtung der Ueberzüge bei Raumtemperatur, durch Einbrennen oder durch Bestrahlung.

Die erfindungsgemässen Korrosionsinhibitoren können auch in wässrigen Systemen ausserhalb der Anstrichstoffe verwendet werden. Dies können rein-wässrige oder teil-wässrige Systeme sein. Beispiele für rein-wässrige Systeme sind Kühlwasser-Systeme, Klimaanlagen, Dampfgeneratoren, Meerwasserverdampfer oder Heizwasser-Kreisläufe. Wenn solche Systeme im Kontakt mit Metallen stehen, benötigen sie den Zusatz eines Korrosionsinhibitors. Die erfindungsgemässen Korrosionsinhibitoren werden in einer Menge von 0,1 bis 50000 ppm (= 0,00001 bis 5%), vorzugsweise 1 bis 500 ppm (= 0,0001 bis 0,05%) zugesetzt. Sie können allein oder in Kombination mit bekannten Korrosionsinhibitoren zugesetzt werden. Bekannte Inhibitoren für wässrige Systeme sind z.B. wasserlösliche Zinksalze, Phosphate, Polyphosphate, Phosphonsäuren und deren Salze wie z.B. Acetodiphosphonsäure, Nitrilotrimethylenphosphonsäure oder Methylaminodimethylenphosphonocarbonsäuren und ihre Salze, beispielsweise solche, wie sie in der DE—OS 2,632,774 beschrieben sind, Phosphonohydroxyessigsäure, 2-Phosphonobutan-1,2,4-tricarbonsäure oder die in GB—A—1,572,406 beschriebenen Säuren. Weitere mitverwendbare bekannte Korrosionsinhibitoren für wässrige Systeme sind Nitrate (z.B. $NaNO_3$), Nitrite (z.B. $NaNO_2$), Molybdate, Wolframate, Silicate, Benztriazol, Tolutriazol und deren Derivate, N-Acrylsarcosine, N-Acyliminodiessigsäuren, Ethanolamine, Fettamine, Polycarbonsäuren wie z.B. Polymerisate und Copolymerisate der Acrylsäure oder der Maleinsäure.

Die wässrigen Systeme können weitere Zusätze enthalten wie Komplexbildner (a.B. Nitrilotriessigsäure), Reduktionsmittel (z.B. Alkalisulfite oder Hydrazin), Antischaum-mittel (z.B. Silicone oder Fettalkohol-Ethylenoxide-Addukte) oder Biocide (z.B. quaternäre Ammoniumverbindungen, Chlorabgebende Verbindungen oder zinnorganische Verbindungen).

Teil-wässrige Systeme, in denen die erfindungsgemässen Korrosionsinhibitoren eingesetzt werden können, sind hydraulische Flüssigkeiten, Gefrierschutzmittel, Metallreinigungs- oder Metallbearbeitungs-Flüssigkeiten, wie sie z.B. beim Polieren, Fräsen, Schneiden, Sägen, Schleifen oder Verformen von Metallen verwendet werden. Diese enthalten meist eine emulgierte ölige Phase, können aber auch wasserlösliche Systeme sein z.B. auf Basis von Polyethylenglykolen. Sie werden oft als Konzentrate in den Handel gebracht, die vor Gebrauch mit Wasser verdünnt werden. Die Zusatzmengen an Korrosionsinhibitor sind dieselben wie bei den reinwässrigen Systemen. Bei den Konzentraten sind Zusatzmengen von 0,5 bis 5% bevorzugt, dies entspricht in den verdünnten Zubereitungen etwa 5 bis 1000 ppm.

Auch in diesem Systemen können die erfindungsgemässen Korrosionsinhibitoren zusammen mit anderen bekannten Inhibitoren verwendet werden. Solche sind z.B. organische Säuren und deren Salze (z.B. Benzoesäure, Dinatrium-sebacat, Triethanolamin-laurat oder Natrium-N-lauroylsarcosinat), organische Stickstoff-Verbindungen (wie z.B. Fettsäuealkanolamide, Imidazole, Oxazoline, Benztriazole, Fettamine, Alkanolamine), anorganische Nitrite (z.B. $NaNO_2$), organische Phosphorverbindungen (z.B. Phosphonsäuren oder Aminophosphonate), anorganische Phosphate (z.B. $NaH_2PO_4$ oder Zinkphosphat) oder organische Schwefelverbindungen und deren Salze (z.B. Salze von Petroleumsulfonaten oder Natrium-mercaptobenzthiazol). Bevorzugt ist die Mitverwendung von Triethanolamin.

Andere gebrächliche Additive für Metallbearbeitungsflüssigkeiten sind Hochdruck-Additive, Emulgatoren und Netzmittel.

Die folgenden Beispiele Illustrieren die vorliegende Erfindung näher. Darin bedeuten Teile Gewichtsteile sofern nicht anders angegeben. Die Temperaturen sind in °C angegeben.

## Beispiel 1: Herstellung der Halbamide

Eine Lösung von 7 g 3-(Benzthiazol-2-ylthio)-propan-1,2-dicarbonsäureanhydrid und 1,8 g n-Butylamin in 50 ml Diethylether with 4 h zum Rückfuss erwärmt. Beim Eindampfen der resultierenden Lösung hinterbleibt ein Gemisch der beiden isomeren Mono(butylamide) als viskoses Oel (Verbindung Nr. 1). NMR-Spektrum (δ $CDCl_3$): 0,92 (t, 2H); 1,18 (m, 4H); 2,70 (m, 2H); 3,16 (m, 3H); 3,50 (m, 2H); 7,10 (m, 2H); 7,58 (m, 2H).

In analoger Weise werden hergestellt (als Isomerengemisch): 3-(Benzthiazol-2-ylthio)-propan-1,2-dicarbonsäure-mono(dodecylamid) (Verbindung Nr. 2) als gelbes Oel. NMR-(δ $CDCl_3$/DMSO): 0,85 (m, 3H); 1,22 (s, 20H); 2,63 (m, 2H); 3,14 (m, 3H); 3,58 (m, 2H); 7,17 (m, 2H); 7,56 (m, 2H).

3-(Benzthiazol-2-ylthio)-propan-1,2-dicarbonsäure-monoanilid (Verbindung Nr. 3) Schmp. 136—9°. NMR (δ $CDCl_3$/DMSO): 2,65 (m, 2H); 3,22 (m, 1H); 3,58 (m, 2H); 7,12 (m, 2H); 7,60 (m, 2H).

(Benzthiazol-2-yl)-bernsteinsäure-mono(butylamid), Schmp. 115—20° (Verbindung Nr. 4). NMR (δ $CDCl_3$/DMSO): 0,85 (m, 3H); 1,35 (m, 4H); 3,14 (m, 4H); 4,90 (m, 1H); 7,35 (m, 2H); 7.81 (m, 2H).

(Benzthiazol-2-yl)-bernsteinsäure-mono(dodecylamid), Schmp. 117—21° (Verbindung Nr. 5). NMR (δ $CDCl_3$/DMSO): 0,85 (in, 3H); 1,22 (m, 20H); 3,15 (m, 4H); 4,84 (m, 1H); 7,25 (m, 2H); 7,42 (m, 2H).

(Benzthiazol-2-yl)-bernsteinsäure-monoanilid (Verbindung Nr. 6), Schmp. 165—8°. NMR (δ $CDCl_3$/DMSO): 3,2 (m, 2H); 4,85 (m, 1H); 7,05 (m, 2H); 7,84 (m, 7H).

## Beispiel 2: Herstellung von Diamiden

In 150 ml absolutes Ethanol wird bei Raumptemperatur trockenes HCl-Gas bis zur Sättigung eingeleitet. Dann werden 50 g 3-(Benthiazol-2-ylthio)-propan-1,2-dicarbonsäure zugegeben und die Mischung 3 Stunden zum Rückfluss erwärmt. Das überschüssige Ethanol wird bei 90° im Vakuum

abdestilliert. Es hinterbleibt der 3-(Benzthiazol-2-ylthio)-propan-1,2-dicarbonsäure-diethylester als gelbe Flüssigkeit.

Ein Gemisch von 18,5 g dieses Esters und 31,1 g Cyclohexylamin wird unter Rühren 12 h auf 95° erwärmt. Nach dem Abkühlen wird Toluol zugegeben. Das ausfallende Kristallisat wird abfilitriert, mit Toluol gewaschen und getrocknet. Das erhaltene 3-(Benzthiazol-2-ylthio)-1,2-dicarbonsäure-di-(cyclohexylamid) schmilzt bei 147—9°C (Verbindung Nr. 7).

In analoger Weise wird aus 18 g des oben genannten Diethylesters und 30 g Butylamin das 3-(Benzthiazol-2-ylthio)-propan-1,2-dicarbonsäure-di(butylamid) als viskose Flüssigkeit erhalten (Verbindung Nr. 8). NMR-Spektrum (δ $CDCl_3$): 1,1 (t, 6H); 1,5 (m, 8H); 2,8 (m, 2H); 3,3 (m 5H); 3,7 (m, 2H); 7,2 (m, 2H); 7,5 (m, 2H).

### Beispiel 3: Herstellung von Imiden

Eine Lösung von 7 g 3-(Benzthiazol-2-ylthio)-propan-1,2-dicarbonsäureanhydrid und 3,2 g n-Octylamin in 100 ml Toluol wird 5 h auf 112° erhitzt bis kein Wasser mehr entsteht. Durch Eindampfen der Lösung erhält man das 3-(Benzthiazol-2-ylthio)-propan-1,2-dicarbonsäure-N-octyl-imid (Verbindung Nr. 9) als braunes Oel. NMR-Spektrum (δ $CDCl_3$): 0,85 (m, 3H); 1,25 (m, 12H); 2,78 (d, 2H); 3,42 (m, 4H); 3,90 (m, 1H); 7,12 (m, 2H); 7,60 (m, 2H).

Analog erhält man aus 6,4 g (Benzthiazol-2-ylthio)-bernsteinsäure-anhydrid und 3,1 g n-Octylamin das (Benzthiazol-2-ylthio)bernsteinsäure-N-octyl-imid (Verbindung Nr. 10) als braunes Oel. NMR-Spektrum (δ $CDCl_3$): 0,85 (m, 3H); 1,22 (m, 12H); 3,18 (d, 2H); 3,55 (t, 2H); 4,30 (t, 1H); 7,12 (m, 2H); 7,54 (m, 2H).

### Beispiel 4: Herstellung von β-(Benzthiazol-2-ylthio)-propionsäureamid

Ein feingepulvertes Gemisch aus 16,8 g 2-Mercaptobenzthiazol und 7,5 g Acrylamid wird bei 45—50° innerhalb einer Stunde unter Rühren in 100 ml 70%ige Schwefelsäure eingetragen. Nach einer weiteren Stunde Rühren bei 45—50° wird das Reaktionsgemisch in Eiswasser gegossen. Die Niederschlag wird abfiltriert, mit Wasser gewaschen und getrocknet. Das erhaltene β-(Benzthiazol-2-ylthio)-propionsäureamid wird aus Ethylacetat umkristallisiert und schmilzt bei 144—5° (Verbindung Nr. 11).

Analyse ($C_{10}H_{10}N_2OS_2$) ber. N 11,76%; S 26,92%
gef. N 11,6%; S 26,6%

### Beispiel 5: Antikorrosiver Lack

Ein Alkydharz-Lack wird durch Zusammenmischen der folgenden Komponenten bereitet:

40 Teile Alphthalat® AM 380 (60%ige Lösung in Xylol) Alkydharz der Fa. Reichhold Albert Chemie AG
4 Teile Eisenoxidrot 225 der Fa. Bayer AG
17,4 Teile Talkum (mikronisiert)
13 Teile Mikronisiertes Calciumcarbonat (Millicarb®, Plüss-Staufer AG)
0,3 Teile Hautverhüttungsmittel Luaktin® (BASF)
0,6 Teile 8%ige Cobaltnaphthenat-Lösung
24,7 Teile Xylol-Ethylglykol-Gemisch 6:60.

Die in den folgenden Tabellen angegebenen Korrosionsinhibitoren werden in einem Teil des Lösungsmittels vorgelöst und dem Lack zugegeben. Der Lack wird 7 Tage mit Glasperlen gemahlen bis zu einer Pigment- und Füllstoffkorngrösse von 15 µm.

Der Lack wird auf sandgestrahlte Stahlbleche von 7 × 13 cm aufgespritzt in einer Schichtdicke, die nach dem Trocknen ca 50 µm beträgt. Nach 7 Tagen Trocknung bei Raumtemperatur werden die Proben 60 Minuten bei 60°C nachgehärtet.

In die gehärtete Lackoberfläche werden zwei kreuzförmige Schnitte von 4 cm Länge bis zum metall eingeschnitten mit Hilfe eines Bonder-Kreuzschnittgerätes. Zum Schutze der Kanten wird auf diese ein Kantenschutzmittel (Icosit®225) aufgebracht.

Die Proben werden nunmehr einem Salzprühtest gemäss ASTM B 117 mit einer Dauer von 600 Stunden unterworfen. Nach jeweils 200 Stunden Bewitterung wird der Zustand des Anstriches beurteilt, und zwar der Blasengrad (gemäss DIN 53 109) am Kreuzschnitt und auf der lackierten Fläche sowie der Rostgrad (gemäss DIN 53210) auf der ganzen Fläche.

Nach Ende des Tests wird der Anstrich durch Behandlung mit konzentrierter Natronlauge entfernt und die Korrosion des Metalls am Kreuzschnitt (gemäss DIN 53 167) sowie über die restliche Fläche beurteilt. Die Beurteilung erfolgt jeweils in einer 6-Stufen-Skala. Die Summe der Bewertung des Anstriches und der

Bewertung der Metalloberfläche ergibt den Korrosionsschutzwert KS. Je höher dieser ist, desto wirkungsvoller ist der getestete Inhibitor.

Korrosionsinhibitoren der Formel

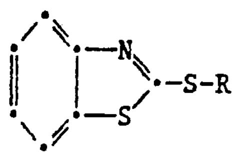

I

| Ver. Nr. | R | Zusatz-Menge | Beurteilung Anstrich | Beurteilung Metall | CP |
|---|---|---|---|---|---|
| 1 | $-CH_2-CH(COOH)-CH_2CONHC_4H_9$ | 2 % | 4,7 | 5,0 | 9,7 |
| | + $-CH_2-CH(CONHC_4H_9)-CH_2COOH$ | 4 % | 4,7 | 4,7 | 9,4 |
| 2 | $-CH_2-CH(COOH)-CH_2CONHC_{12}H_{25}$ | 2 % | 4,5 | 4,6 | 9,1 |
| | + $-CH_2-CH(CONHC_{12}H_{25})-CH_2COOH$ | 4 % | 4,0 | 3,3 | 7,3 |
| 3 | $-CH_2-CH(COOH)-CH_2-CONHC_6H_5$ | 2 % | 3,4 | 1,5 | 4,9 |
| | + $-CH_2-CH(CONHC_6H_5)-CH_2-COOH$ | 4 % | 3,0 | 1,7 | 4,7 |
| 4 | $-CH(COOH)-CH_2CONHC_4H_9$ | 2 % | 2,8 | 1,5 | 4,3 |
| | + $-CH(CONHC_4H_9)-CH_2COOH$ | 4 % | 2,6 | 0,6 | 3,2 |
| 5 | $-CH(COOH)-CH_2CONHC_{12}H_{25}$ | 2 % | 3,6 | 3,8 | 7,4 |
| | + $-CH(CONHC_{12}H_{25})-CH_2COOH$ | 4 % | 1,6 | 0,6 | 2,2 |
| 6 | $-CH(COOH)-CH_2CONHC_6H_5$ | 2 % | 4,5 | 5,0 | 9,5 |
| | + $-CH(CONHC_6H_5)-CH_2COOH$ | 4 % | 2,5 | 0,6 | 3,1 |
| 7 | $-CH_2-CH(CONHC_6H_{11}\text{-cyclo})-CH_2CONHC_6H_{11}\text{-cyclo}$ | 2 % | 4,1 | 4,0 | 8,1 |
| | | 4 % | 3,8 | 3,3 | 7,1 |

| Ver. No. | R | Zusatz-Menge | Beurteilung | | CP |
|---|---|---|---|---|---|
| | | | Anstrich | Metall | |
| 8 | $-CH_2-CH-CH_2CONHC_4H_9$<br>$CONHC_4H_9$ | 2 %<br>4 % | 4,9<br>4,5 | 3,5<br>1,7 | 8,4<br>6,2 |
| 9 | 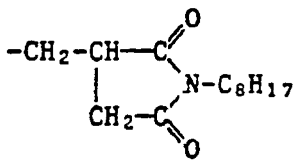 | 2 %<br>4 % | 3,7<br>2,9 | 3,8<br>1,5 | 7,5<br>4,4 |
| 10 | 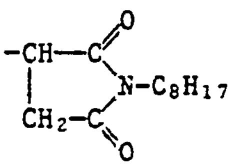 | 2 %<br>4 % | 4,4<br>4,7 | 3,8<br>4,3 | 8,2<br>9,0 |
| 11 | $-CH_2-CH_2CONH_2$ | 2 %<br>4 % | 4,3<br>3,4 | 4,8<br>3,9 | 9,1<br>7,3 |
| - | ohne Korrosionsinhibitor | - | 1,6 | 0,8 | 2,4 |

**Patentansprüche**

1. Verwendung von Amiden, Imiden oder Nitrilen von aliphatischen oder cycloaliphatischen Mono-, Di-, Tri- oder Tetracarbonsäuren, die in ihrem (cyclo-)aliphatischen Rest durch eine oder mehrere Gruppen der Formel I substituiert sind,

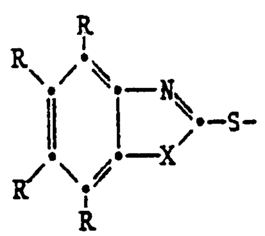

I

worin X Schwefel bedeutet und jedes R unabhängig voneinander Wasserstoff, Alkyl, Halogenalkyl, Alkoxy, Alkylthio, Alkylsulfonyl, Cycloalkyl, Phenyl, Alkylphenyl, Phenylalkyl, Halogen, —CN, —$NO_2$, —COOH, —COOAlkyl, —OH oder eine primäre, sekundäre oder tertiäre Amino- oder Carbamoylgruppe bedeutet, sowie nicht-toxische Basen-Additionssalze dieser Verbindungen, soweit sie Carboxylgruppen enthalten, als Korrosionsinhibitoren.

2. Verwendung eines Amides gemäss Anspruch 1.

3. Verwendung eines Halbamides einer Di, Tri- oder Tetracarbonsäure gemäss Anspruch 1.

4. Verwendung gemäss Anspruch 1 einer Verbindung mit einer Gruppe der Formel I, worin einer der Substituenten R Wasserstoff $C_1$—$C_4$-Alkoxy oder eine Aminogruppe ist und die anderen drei R Wasserstoff sind.

5. Verwendung gemäss Anspruch 4, wobei alle vier R Wasserstoff sind.

6. Verwendung gemäss Anspruch 1 einer Verbindung der Formel II

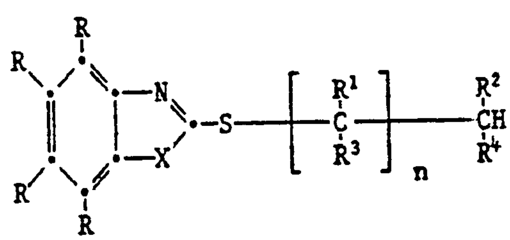

II

oder eines nicht-toxischen Basenadditions-Salzes davon, worin X und R die in Anspruch 1 gegebene Bedeutung haben, n null oder 1 ist und $R^1$, $R^2$, $R^3$ und $R^4$ unabhängig voneinander Wasserstoff, Alkyl, Hydroxyalkyl, Halogenalkyl, Cyanoalkyl, Alkoxyalkyl, unsubstituiertes oder im Phenylkern durch Halogen, Nitro, Alkyl, Alkoxy, Carboxy oder Hydroxy substituiertes Phenyl oder Phenylalkyl bedeutet oder eine Gruppe —COOH, —CN oder —$CONZ_1Z_2$ oder durch 1, 2 oder 3 Gruppen —COOH, —CN oder —$CONZ_1Z_2$ substituiertes Alkyl sind, wobei $Z_1$ und $Z_2$ unabhängig voneinander Wasserstoff oder $C_1$—$C_{18}$-Alkyl, das durch ein oder mehrere O- oder S-Atome oder NR°-Gruppen unterbrochen sein kann (R° = $C_1$—$C_{18}$-Alkyl, $C_5$—$C_8$-Cycloalkyl, Phenyl, Naphthyl, $C_7$—$C_9$-Phenylalkyl oder $C_7$—$C_{18}$-Alkylphenyl) oder durch —SH, —$NH_2$, —COOH, —COOR°, —$CONH_2$, —CN oder Halogen substituiert sein kann, bedeuten oder $C_2$—$C_{10}$-Hydroxyalkyl, das durch eine oder mehrere NR°-Gruppen oder O-Atome unterbrochen sein kann, bedeuten oder $C_2$—$C_{18}$-Alkenyl oder $C_3$—$C_{12}$-Cycloalkyl, das durch $C_1$—$C_4$-Alkyl, —OH, —SH, —COOR°, —$CONH_2$, —CN oder Halogen substituiert sein kann, bedeuten oder $C_7$—$C_9$-Phenylalkyl, $C_7$—$C_{18}$-Alkylphenyl oder $C_6$—$C_{10}$-Aryl, das durch $C_1$—$C_{12}$-Alkoxy, $C_1$—$C_{12}$-Alkylthio, —COOH, —OH, —$NO_2$ oder Halogen substituiert sein kann bedeuten oder $Z_1$ und $Z_2$ zusammen gegebenenfalls verzweigtes $C_3$—$C_7$-Alkylen sind, das durch O, S oder NR° unterbrochen sein kann, oder einer Verbindung der Formel II, die eine Gruppe der Formel III oder IIIa enthalt

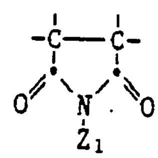 III 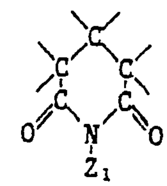 IIIa

oder einer Verbindung der Formel II, worin n = 1 ist und $R^1$ und $R^2$ zusammen oder $R^1$ und $R^2$ zusammen eine gegebenenfalls verzweigte Alkenylgruppe bilden, die durch 1 oder 2 Gruppen —COOH oder —$CONZ_1Z_2$ substituiert sein kann,

oder einer Verbindung der Formel II, worin n = 1 ist und $R^1$ und $R^2$ zusammen eine direkte Bildung bilden, wobei der Rest IV

$$—[C(R^1)(R^3)—]_n CH(R^2)(R^4) \qquad \text{IV}$$

mindestens eine Gruppe —$CONZ_1Z_2$ oder —CN oder eine Gruppe der Formel III oder IIIa enthält.

7. Verwendung gemäss Anspruch 6 einer Verbindung der Formel II, worin $R^1$, $R^2$, $R^3$ und $R^4$ Wasserstoff, $C_1$—$C_4$-Alkyl, —CN, —COOH, —$CONZ_1Z_2$ oder durch —CN, —COOH oder —$CONZ_1Z_2$ substituiertes Alkyl bedeuten.

8. Verwendung gemäss Anspruch 7 einer Verbindung der Formel II, worin $R^4$ eine Gruppe —COOH oder —$CONZ_1Z_2$ oder durch —COOH oder —$CONZ_1Z_2$ substituiertes $C_1$—$C_4$-Alkyl ist.

9. Verwendung gemäss Anspruch 7 einer Verbindung der Formel II, worin n = 1 ist und mindestens zwei der Reste $R^1$, $R^2$, $R^3$ und $R^4$ eine Gruppe —COOH, —$CONZ_1Z_2$ oder durch —COOH oder —$CONZ_1Z_2$ substituiertes Alkyl sind.

10. Verwendung gemäss Anspruch 7 einer Verbindung der Formel II worin entweder zwei Gruppen —$CONZ_1Z_2$ oder eine Gruppe —COOH und eine Gruppe —$CONZ_1Z_2$ an zwei benachbarten C-Atomen gebunden sind.

11. Verwendung gemäss Anspruch 6 einer Verbindung der Formel II, worin $Z_1$ und $Z_2$ unabhängig voneinander Wasserstoff, $C_1$—$C_{12}$-Alkyl, $C_2$—$C_4$-Hydroxyalkyl, $C_3$—$C_{12}$-Alkoxalkyl, Cyclohexyl, Benzyl, Phenyl, Tolyl oder Naphthyl oder $Z_1$ und $Z_2$ zusammen Tetramethylen, Pentamethylen oder 3-Oxapentamethylen sind.

12. Antikorrosiver Anstrichstoff enthaltend als Korrosionsinhibitor 0,1 bis 20 Gew.-% vorzugsweise 0,5 bis 5 Gw.-% (bezogen auf den Feststoffgehalt des Anstrichstoffes) eines Amides, Imides oder Nitrils einer aliphatischen oder cycloaliphatischen Mono-, Di- Tri- oder Tetracarbonsäure, die in ihrem (cyclo)aliphatischen Rest durch eine oder mehrere Gruppen der Formel I substituiert ist,

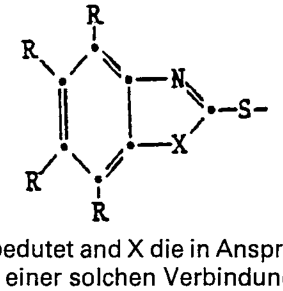 I

worin X Sauerstoff Schwefel oder NH bedutet and X die in Anspruch 1 gegebene Bedeutung hat, oder eines nicht-toxischen Basenadditions-Salzes einer solchen Verbindung, soweit sie eine Carboxylgruppe enthält.

13. Anstrichstoff gemäss Anspruch 12 auf nicht-wässriger Basis, der als Bindemittel ein Epoxidharz,

Polyurethan, Aminoplast, Acrylharz, Polyesterharz, Alkydharz, Phenolharz, Vinylharz, chloriertes Harz, Styrol-Copolymerisat, Celluloseester, trockenes Oel oder ein Gemisch solcher Harze enthält.

14. Anstrichstoff gemäss Anspruch 12 auf wässriger Basis, der als Bindemittel ein Alkydharz, einen Polyester, ein Acrylharz, Polyurethan, Epoxidharz, Phenolharz, Aminoplastharz, Vinylharz, Styrolharz oder ein Gemisch solcher Harze enthält.

15. Anstrichstoff gemäss Anspruch 12, enthaltend ausser dem Bindemittel und dem Korrosionsinhibitor ein Pigment, einen Füllstoff oder sonstige für Anstrichstoffe übliche Zusätze.

16. Anstrichstoff gemäss Anspruch 15, enthaltend einen basischen Füllstoff oder ein basisches Pigment.

17. Verwendung eines antikorrosiven Anstrichstoffes gemäss Anspruch 12 als Primer auf metallischen Substraten.

18. Verwendung gemäss Anspruch 17 als Elektrotauchlack.

19. Antikorrosives wässriges System ausserhalb der Verwendung als Anstrichstoff, enthaltend als Korrosionsinhibitor 0,1 bis 50 000 ppm, vorzugsweise 1 bis 500 ppm, eines Amides, Imides oder Nitrils einer aliphatischen oder cycloaliphatischen Mono-, Di-, Tri- oder Tetracarbonsäure, die in ihrem (cyclo)aliphatischen Rest durch eine Gruppe der Formel I substituiert ist,

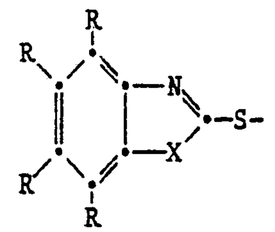

I

worin R und X die in Anspruch 1 gegebene Bedeutung haben, oder eines nicht-toxischen Basenadditions-Salzes einer solchen Verbindung, die eine Carboxylgruppe enthält.

20. Kühlwassersystem, Klimaanlage, Dampfgenerator, Meerwasserverdampfer, Heizwassersystem, Gefrierschutzmittel, hydraulische Flüssigkeit, wässriges Metallreinigungs- oder Metallbearbeitungsmittel gemäss Anspruch 19.

21. Wässriges System gemäss Anspruch 20, enthaltend ausser dem in Anspruch 16 definierten Korrosionsinhibitor einen oder mehrere andere Korrosionsinhibitoren, Komplexbildner, Reduktionsmittel, Antischaum-mittel, Biocide, Emulgatoren, Netzmittel oder Hochdruck-Additive.

22. Verbindungen der Formel II,

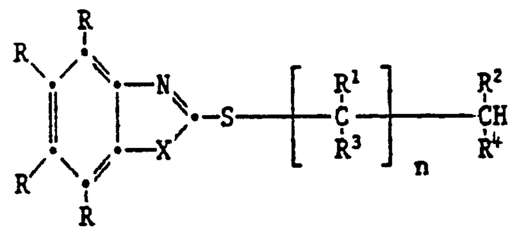

II

worin X Schwefel bedeutet und jedes R unabhängig voneinander Wasserstoff, Alkyl, Halogenalkyl, Alkoxy, Alkylthio, Alkylsulfonyl, Cycloalkyl, Phenyl, Alkylphenyl, Phenylalkyl, Halogen, —CN, —$CO_2$, —COOH, —COOAlkyl, —OH oder eine primäre, sekundäre oder tertiäre Amino- oder Carbamoylgruppe bedeutet, n null oder 1 ist und $R^1$, $R^2$, $R^3$ und $R^4$ unabhängig voneinander Wasserstoff, Alkyl, Hydroxyalkyl, Halogenalkyl, Cyanoalkyl, Alkoxyalkyl, unsubstituiertes oder im Phenylkern durch Halogen, Nitro, Alkyl, Alkoxy, Carboxy oder Hydroxy substituiertes Phenyl oder Phenylalkyl bedeutet oder eine Gruppe —COOH, —CN oder —$CONZ_1Z_2$ oder durch 1, 2 oder 3 Gruppen —COOH, —CN oder —$CONZ_1Z_2$ substituiertes Alkyl sind, wobei $Z_1$ und $Z_2$ unabhängig voneinander Wasserstoff oder $C_1$—$C_{18}$-Alkyl, das durch ein oder mehrere O- oder S-Atome oder NR°-Gruppen unterbrochen sein kann (R° = $C_1$—$C_{18}$-Alkyl, $C_5$—$C_8$-Cycloalkyl, Phenyl, Naphthyl, $C_7$—$C_9$-Phenylalkyl oder $C_7$—$C_{18}$-Alkylphenyl) oder durch —SH, —$NH_2$, —COOH, —COOR°, —$CONH_2$, —CN oder Halogen substituiert sein kann, bedeuten oder $C_2$—$C_{10}$-Hydroxyalkyl, das durch eine oder mehrere NR°-Gruppen oder O-Atome unterbrochen sein kann, bedeuten oder $C_2$—$C_{18}$-Alkenyl oder $C_3$—$C_{12}$-Cycloalkyl, das durch $C_1$—$C_4$-Alkyl, —OH, —SH, —COOR°, —$COHN_2$, —CN oder Halogen substituiert sein kann, bedeuten oder $C_7$—$C_9$-Phenylalkyl, $C_7$—$C_{18}$-Alkylphenyl oder $C_6$—$C_{10}$-Aryl, das durch $C_1$—$C_{12}$-Alkoxy, $C_1$—$C_{12}$-Alkylthio, —COOH, —OH, —$NO_2$ oder Halogen substituiert sein kann, bedeuten oder $Z_1$ und $Z_2$ zusammen gegebenenfalls verzweigtes $C_3$—$C_7$-Alkylen sind, das durch O, S oder NR° unterbrochen sein kann, und die im Rest

$$-\!\!-\!\![C(R^1)(R^3)-\!\!-]_n\,CH(R^2)(R^4)$$

2, 3 oder 4 Amidgruppen —$CONZ_1Z_2$ enthalten.

23. Verbindungen der Formel II,

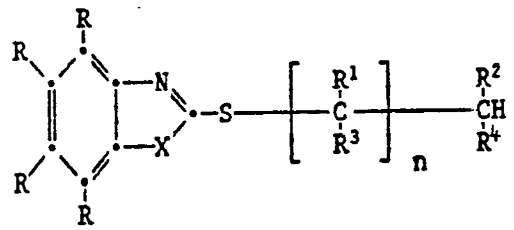

II

worin X, R, $R^1$, $R^2$, $R^3$ und $R^4$ die in Anspruch 22 gegebene Bedeutung haben und die im Rest

$$-\!\!\left[C(R^1)(R^3)-\right]_n CH(R^2)(R^4) \qquad \text{IV}$$

mindestens eine Gruppe —$CONZ_1Z_2$ und mindestens eine Gruppe —COOH enthalten.

24. Verbindungen der Formel II

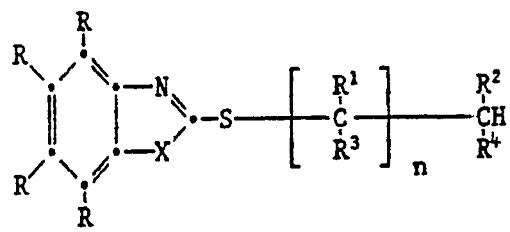

II

worin X, R, $R^1$, $R^2$, $R^3$ und $R^4$ die in Anspruch 22 gegebene Bedeutung haben und die eine oder zwei Gruppen der Formel III oder IIIa

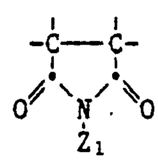

III

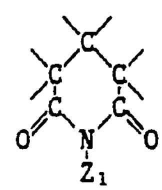

IIIa

enthalten.

25. Verbindungen der Formel II,

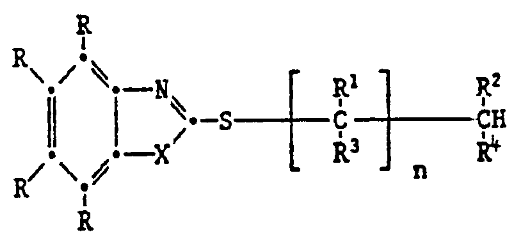

II

worin X, R, $R^1$, $R^2$, $R^3$ und $R^4$ die in Anspruch 22 gegebene Bedeutung haben und die im Rest

$$-\!\!\left[C(R^1)(R^3)-\right]_n CH(R^2)(R^4)$$

2, 3 oder 4 Cyanogruppen enthalten.

**Revendications**

1. Application, comme inhibiteurs de corrosion, d'amides, d'imides ou de nitriles d'acides monocarboxyliques, dicarboxyliques, tricarboxyliques ou tétracarboxyliques aliphatiques ou

cycloaliphatiques dont le radical aliphatique ou cycloaliphatique porte un ou plusieurs radicaux, de préférence un seul, répondant à la formule I:

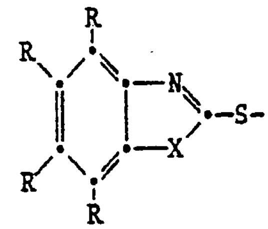

I

dans laquelle: X représente le soufre et les R représentent chacun, indépendamment les uns des autres, l'hydrogène, un radical alkyle, halogénoalkyle, alcoxy, alkylthio, alkylsulfonyl, cycloalkyle, phényle, alkylphényle ou phénylalkyle, unhalogène, un radical —CN, —$NO_2$, —COOH, —COOAlkyl ou —OH ou un radical amino ou carbamoyle primaire, secondaire ou tertiaire, ainsi que de sels non toxiques que ces composés, dans la mesure où ils contiennent des radicaux carboxy, forment par addition avec des bases.

2. Application d'un amide selon la revendication 1.

3. Application d'un semi-amide d'un acide dicarboxylique, tricarboxylique ou tétracarboxylique selon la revendication 1.

4. Application selon la revendication 1 d'un composé contenant un radical de formule I dans lequel l'un des symboles R représente l'hydrogène, un alcoxy en $C_1$—$C_4$ ou un amino et les trois autres R représentent chacun l'hydrogène.

5. Application selon la revendication 4 selon laquelle les quatre symboles R représentent chacun l'hydrogène.

6. Application selon la revendication 1 d'un composé répondant à la formule II:

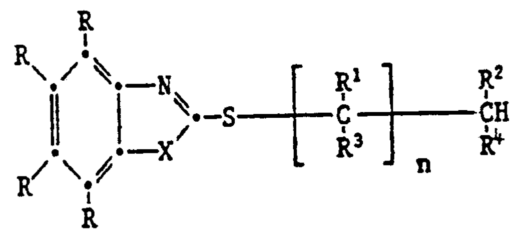

II

(ou d'un sel d'addition non toxique d'un tel composé avec une base) dans laquelle X et R ont les significations qui leur ont été données à la revendication 1, n est égal à 0 ou à 1, $R^1$, $R^2$, $R^3$ et $R^4$ représentent chacun, indépendamment les uns des autres, l'hydrogène, un alkyle, un hydroxyalkyle, un halogénoalkyle, un cyanalkyle, un alcoxyalkyle, un radical phényle ou phénylalkyle non substitué ou à noyau phényle porteur d'un halogène ou d'un radical nitro, alkyle, alcoxy, carboxy ou hydroxy, un radical —COOH, —CN ou —$CONZ_1Z_2$, ou un alkyle porteur d'un, de deux ou de trois radicaux —COOH, —CN ou —$CONZ_1Z_2$, et $Z_1$ et $Z_2$ représentent chacun, indépendamment l'un de l'autre, l'hydrogène, un alkyle en $C_1$—$C_{18}$ éventuellement interrompu une ou plusieurs fois par une atome O, un atome S ou un radical NR° (le symbol R° désignant un alkyle en $C_1$—$C_{18}$, un cycloalkyle en $C_5$—$C_8$, un phényle, un naphtyle, un phénylalkyle en $C_7$—$C_9$ ou un alkylphényle en $C_7$—$C_{18}$) ou éventuellement porteur d'un radical —SH, —$NH_2$, —COOH, —COOR°, —$CONH_2$ ou —CN ou d'un halogène, un hydroxy-alkyle en $C_2$—$C_{10}$ éventuellement interrompu par un ou plusieurs radicaux NR° ou atomes d'oxygène, un radical alcényle en $C_2$—$C_{18}$ ou cycloalkyle en $C_3$—$C_{12}$ éventuellement porteur d'un alkyle en $C_1$—$C_4$, d'un radical —OH, —SH, —COOR°, —$CONH_2$ ou —CN ou d'un halogène, ou un radical phénylalkyle en $C_7$—$C_9$, alkylphényle en $C_7$—$C_{18}$ ou aryle en $C_6$—$C_{10}$ éventuellement porteur d'un alcoxy en $C_1$—$C_{12}$, d'un alkylthio en $C_1$—$C_{12}$, d'un radical —COOH, —OH, —$NO_2$ ou d'un halogène, ou encore $Z_1$ et $Z_2$ représentent ensemble un radical alkylène en $C_3$—$C_7$ éventuellement ramifié qui peut être interrompu par O, par S ou par NR°, ou d'un composé de formule II qui contient un radical de formule III ou de formule IIIa:

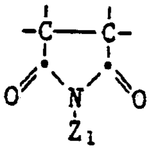

III

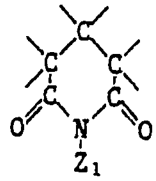

IIIa

ou d'un composé de formule II dans lequel n est égal à 1 et $R^1$ et $R^2$ ensemble, ou $R^1$ et $R^3$ ensemble, représentent un alkylène éventuellement ramifié qui peut porter un ou deux radicaux —COOH ou

—$CONZ_1Z_2$, ou d'un composé de formule II dans lequel n est égal à 1 et $R^1$ et $R^2$ représentent ensemble une liaison directe, le radical IV:

$$—[C(R^1)(R^3)—]_n CH(R^2)(R^4) \qquad IV$$

contenant au moins un radical —$CONZ_1Z_2$ ou —CN ou un radical de formule III ou IIIa.

7. Application selon la revendication 6 d'un composé de formule II dans lequel $R^1$, $R^2$, $R^3$ et $R^4$ représentent chacun l'hydrogène, un alkyle en $C_1$—$C_4$, un —CN, un —COOH, un —$CONZ_1Z_2$ ou un alkyle porteur d'un radical —CN, —COOH ou —$CONZ_1Z_2$.

8. Application selon la revendication 7 d'un composé de formule II dans lequel $R^4$ représente un radical —COOH ou —$CONZ_1Z_2$ ou un alkyle en $C_1$—$C_4$ porteur d'un radical —COOH ou —$CONZ_1Z_2$.

9. Application selon la revendication 7 d'un composé de formule II dans lequel n est égal à 1 et au moins deux des symboles $R^1$, $R^2$, $R^3$ et $R^4$ représentent un radical —COOH, un radical —$CONZ_1Z_2$ ou un radical alkyle porteur d'un radical —COOH ou —$CONZ_1Z_2$.

10. Application selon la revendication 9 d'un composé de formule II dans lequel ou bien deux radicaux —$CONZ_1Z_2$, ou bien un radical —COOH et un radical —$CONZ_1Z_2$ sont liés à deux atomes de carbone voisins.

11. Application selon la revendication 6 d'un composé de formule II dans lequel $Z_1$ et $Z_2$ représentent chacun, indépendamment l'une de l'autre, l'hydrogène, un alkyle en $C_1$—$C_{12}$, un hydroyalkyle en $C_2$—$C_4$, un alcoxy alkyle en $C_3$—$C_{12}$, un cyclohexyle, un benzyle, un phényle, un tolyle ou un naphtyle ou $Z_1$ et $Z_2$ représentent ensemble un radical tétraméthylène, pentaméthylène ou oxa-3 pentaméthylène.

12. Peinture anti-corrosion qui contient, comme inhibiteur de corrosion, de 0,1 à 20% en poids, de préférence de 0,5 à 5% en poids (par rapport à la matière solide de la peinture) d'un amide, d'un imide ou d'un nitrile d'un acide monocarboxylique, dicarboxylique, tricarboxylique ou tétracarboxylique aliphatique ou cycloaliphatique porte un ou plusieurs radicaux répondant à la formule I:

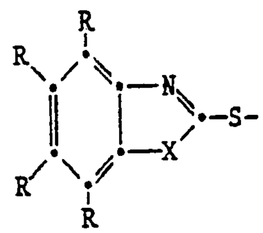

I

dans laquelle R et X ont les significations qui leur ont été données à la revendication 1, ou d'un sel d'adition non toxique formé par un tel composé avec une base dans la mesure où ledit composé renferme un radical carboxy.

13. Peinture selon la revendication 12 à base non aqueuse qui contient, comme liant, une résine époxydique, un polyuréthanne, un aminoplaste, une résine acrylique, une résine de polyester, une résine alkyde, une résine phénolique, une résine vinylique, une résine chlorée, un copolymère du styrène, un ester de la cellulose, une huile siccative ou un mélange de telles résines.

14. Peinture selon la revendication 12 à base aqueuse qui contient, comme liant, une résine alkyde, un polyester, une résine phénolique, un polyuréthanne, une résine phénolique, une résine aminoplaste, une résine vinylique, une résine de styrène ou un mélange de telles résines.

15. Peinture selon la revendication 12 qui, en plus ly liant et de l'inhibiteur de corrosion, contient un pigment, une charge ou d'autres additifs usuels pour peintures.

16. Peinture selon la revendication 15 qui contient une charge basique ou un pigment basique.

17. Application d'une peinture anti-corrosion selon la revendication 12 comme revêtement de fond sur substrats métalliques.

18. Application selon la revendication 17 comme peinture applicable par trempage électrophorétique.

19. Système aqueux anti-corrosion, applicable en dehors du domaine des peintures, qui contient, comme inhibiteur de corrosion, de 0,1 à 50000 ppm, de préférence de 1 à 500 ppm, d'un amide, d'un amide ou d'un nitrile d'un acide monocarboxylique, dicarboxylique, tricarboxylique ou tétracarboxylique aliphatique ou cycloaliphatique dont le radical aliphatique ou cycloaliphatique porte un radical répondant à la formule I:

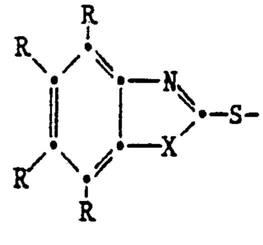

I

dans lequelle R et X ont les significations qui leur ont été données à la revendication 1, ou d'un sel d'addition non toxique formé par un tel composé avec une base dans la mesure où ledit composé renferme un radical carboxy.

20. Système d'eau de refroidissement, installation de climatisation, générateur de vapeur, évaporateur d'eau de mer, système d'eau de chauffage, produit anti-gel, liquide hydraulique et produit aqueux pour le nettoyage ou l'usinage de métaux, selon la revendication 19.

21. Système aqueux selon la revendication 20 qui, en plus de l'inhibiteur de corrosion défini à la revendication 16, contient un ou plusieurs autres inhibiteurs de corrosion, des formateurs de complexes, des réducteurs, des anti-mousses, des biocides, des émulsionnants, des mouillants ou des additifs hautes pressions.

22. Composés répondant à la formule II:

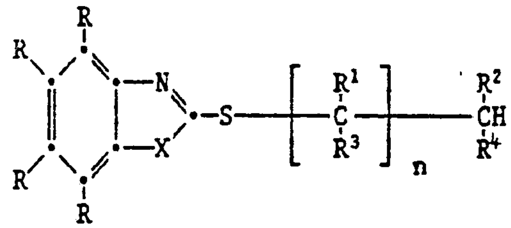

II

dans laquelle X représente le soufre, les symboles R représentent chacun, indépendamment les uns des autres, l'hydrogène, un radical alkyle, halogénoalkyle, alcoxy, alkylthio, alkylsulfonyle, cycloalkyle, phényle, alkylphényle ou phénylalkyle, un halogène, un radical —CN, $—NO_2$, —COOH, —COOalkyl ou —OH, ou un radical amino ou carbamoyle primaire, secondaire ou tertiaire, n est égal à 0 ou à 1, $R^1$, $R^2$, $R^3$ et $R^4$ représentent chacun, indépendamment les uns des autres, l'hydrogène, un alkyle, un hydroxyalkyle, un halogéno-alkyl, un cyanalkyle, un alkoxyalkyle, un radical phényle ou phénylalkyle non substitué ou à noyau phényle porteur d'un halogène ou d'un radical nitro, alkyle, alcoxy, carboxy ou hydroxy, un radical —COOH, —CN ou $—CONZ_1Z_2$, ou un alkyle porteur d'un, de deux ou de trois radicaux —COOH, —CN ou $—CONZ_1Z_2$, et $Z_1$ et $Z_2$ représentent chacun, indépendamment l'un de l'autre, l'hydrogène, un alkyle en $C_1—C_{18}$ éventuellement interrompu une ou plusieurs fois par un atome O, un atome S ou un radical NR° (le symbole R° désignant un alkyle en $C_1—C_{18}$, un cycloalkyle en $C_5—C_8$, un phényle, un naphtyle, un phényl-alkyle en $C_7—C_9$ ou un alkylphényle en $C_7—C_{18}$) ou éventuellement porteur d'un radical —SH, $—NH_2$, —COOH, —COOR°, $—CONH_2$ ou —CN ou d'un halogène, un hydroxy-alkyle en $C_2—C_{10}$ éventuellement interrompu par un ou plusieurs radicaux NR° ou atomes d'oxygène, un radical alcényle en $C_2—C_{18}$ ou cycloalkyle en $C_3—C_{12}$ éventuellement porteur d'un alkyle en $C_1—C_4$, d'un radical —OH, —SH, —COOR°, $CONH_2$ ou —CN ou d'un halogène, ou un radical phénylalkyle en $C_7—C_9$, alkylphényle en $C_7—C_{18}$ ou aryle en $C_6—C_{10}$ éventuellement porteur d'un alcoxy en $C_1—C_{12}$, d'un alkylthio en $C_1—C_{12}$, d'un radical —COOH, —OH, $—NO_2$ ou d'un halogène, ou encore $Z_1$ et $Z_2$ représentent ensemble un radical alkylène en $C_3—C_7$ éventuellement ramifié qui peut être interrompu par O, par S ou par NR°, et qui contiennent, dans le radical:

$$—[C(R^1)(R^3)—]_n CH(R^2)(R^4)$$

2, 3 ou 4 radicaux àmides $—CONZ_1Z_2$.

23. Composés répondant à la formule II:

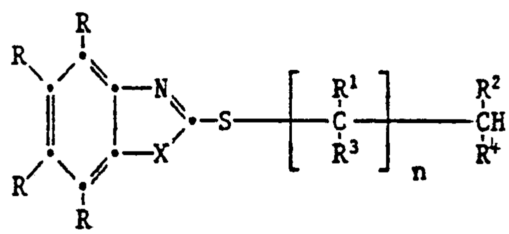

II

dans laquelle X, R, $R^1$, $R^2$, $R^3$ et $R^4$ ont les significations qui leur ont été données à la revendication 22 et qui contiennent, dans le radical:

$$—[C(R^1)(R^3)—]_n CH(R^2)(R^4) \qquad IV$$

au moins un radical —$CONZ_1Z_2$ et au moins un radical —COOH.

24. Composés répondant à la formule II:

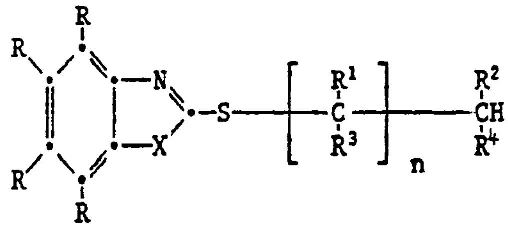

II

dans laquelle X, R, $R^1$, $R^2$, $R^3$ et $R^4$ ont les significations qui leur ont été données à la revendication 22 et qui contiennent un ou deux radicaux de formule III ou IIIa:

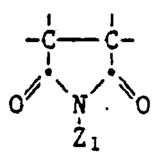

III

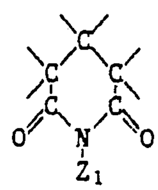

IIIa

25. Composés répondant à la formule II:

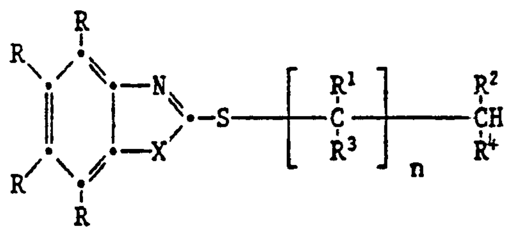

II

dans laquelle X, R, $R^1$, $R^2$, $R^3$ et $R^4$ ont les significations qui leur ont été données à la revendication 22 et qui contiennent, dans le radical:

$$-\!\!-[C(R^1)(R^3)-\!\!-]_n\, CH(R^2)(R^4)$$

2, 3 ou 4 radicaux cyano.

## Claims

1. Use of amides, imides or nitriles of aliphatic or cycloaliphatic mono-, di-, tri- or tetra-carboxylic acids which are substituted in the (cyclo-)aliphatic radical by one or more groups of the formula I

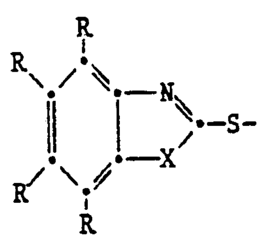

I

in which X is sulphur and each R independently of one another is hydrogen, alkyl, haloalkyl, alkoxy, alkylthio, alkylsulphonyl, cycloalkyl, phenyl, alkylphenyl, phenylalkyl, halogen, —CN, —$NO_2$, —COOH, —COOalkyl, —OH or a primary, secondary or tertiary amino or carbamoyl group, and also non-toxic base addition salts of these compounds, in as far as they contain carboxyl groups, as corrosion inhibitors.

2. Use of an amide according to claim 1.

3. Use of a half-amide of a di-, tri- or tetra-carboxylic acid according to claim 1.

4. Use according to claim 1 of a compound having a group of the formula I in which one of the substituents R is hydrogen, $C_1$—$C_4$alkoxy or an amino group and the other three substituents R are hydrogen.

5. Use according to claim 4 where all four substituents R are hydrogen.

6. Use according to claim 1 of a compound of the formula II

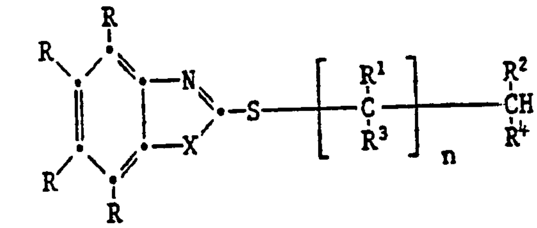

II

or of a non-toxic base addition salt thereof in which X and R are as defined in claim 1, n is 0 or 1 and $R^1$, $R^2$, $R^3$ and $R^4$ independently of one another are hydrogen, alkyl, hydroxyalkyl, haloalkyl, cyanoalkyl, alkoxyalkyl, or phenyl or phenylalkyl each of which is unsubstituted or substituted in the phenyl nucleus by halogen, nitro, alkyl, alkoxy, carboxyl or hydroxyl, or a —COOH, —CN or —$CONZ_1Z_2$ group, or alkyl substituted by 1, 2 or 3 —COOH, —CN or —$CONZ_1Z_2$ groups, $Z_1$ and $Z_2$ being independently of one another hydrogen or $C_1$—$C_{18}$alkyl which may be interrupted by one or more O and S atoms or NR° groups (R° = $C_1$—$C_{18}$alkyl, $C_5$—$C_8$cycloalkyl, phenyl, naphthyl, $C_7$—$C_9$phenylalkyl or $C_7$—$C_{18}$alkylphenyl) or may be substituted by —SH, —$NH_2$, —COOH, —COOR°, —$CONH_2$, —CN or halogen, or are $C_2$—$C_{10}$hydroxyalkyl which may be interrupted by one or more NR° groups or O atoms, or are $C_2$—$C_{18}$alkenyl or $C_3$—$C_{12}$cycloalkyl which may be substituted by $C_1$—$C_4$alkyl, —OH, —SH, —COOR°, —$CONH_2$, —CN or halogen, or are $C_7$—$C_9$phenylalkyl, $C_7$—$C_{18}$alkylphenyl or $C_6$—$C_{10}$aryl which may be substituted by $C_1$—$C_{12}$alkoxy, $C_1$—$C_{12}$alkylthio, —COOH, —OH, —$NO_2$ or halogen, or $Z_1$ and $Z_2$ together are optionally branched $C_3$—$C_7$alkylene which may be interrupted by O, S or NR°, or of a compound of the formula II which contains a group of the formula III or IIIa

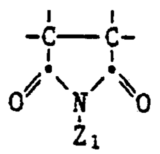

III

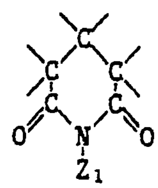

IIIa

or of a compound of the formula II in which n is 1 and $R^1$ and $R^2$ together or $R^1$ and $R^3$ together form an optionally branched alkylene group which may be substituted by 1 or 2 —COOH or $CONZ_1Z_2$ groups, or of a compound of the formula II in which n is 1 and $R^1$ and $R^2$ together form a direct bond, where the radical IV

$$—\!\!\left[C(R^1)(R^3)—\right]_n CH(R^2)(R^4) \qquad \text{IV}$$

contains at least one $CONZ_1Z_2$ or —CN group or a group of the formula III or IIIa.

7. Use according to claim 6 of a compound of the formula II in which $R^1$, $R^2$, $R^3$ and $R^4$ are halogen, $C_1$—$C_4$alkyl, —CN, —COOH, $CONZ_1Z_2$ or alkyl substituted by —CN, —COOH or $CONZ_1Z_2$.

8. Use according to claim 7 of a compound of the formula II in which $R^4$ is a —COOH or $CONZ_1Z_2$ group or $C_1$—$C_4$alkyl substituted by —COOH or $CONZ_1Z_2$.

9. Use according to claim 7 of a compound of the formula II in which n is 1 and at least two of the radicals $R^1$, $R^2$, $R^3$ and $R^4$ are a —COOH, $CONZ_1Z_2$ group or alkyl substituted by —COOH or $CONZ_1Z_2$.

10. Use according to claim 9 of the formula II in which either two $CONZ_1Z_2$ groups or one —COOH group and one $CONZ_1Z_2$ group are bound on two adjacent carbon atoms.

11. Use according to claim 6 of a compound of the formula II in which $Z_1$ and $Z_2$ independently of one another are hydrogen, $C_1$—$C_{12}$alkyl, $C_2$—$C_4$hydroxyalkyl, $C_3$—$C_{12}$alkoxyalkyl, cyclohexyl, benzyl, phenyl, tolyl or naphthyl or $Z_1$ and $Z_2$ together are tetramethylene, pentamethylene or 3-oxapentamethylene.

12. Anticorrosive coating composition containing as corrosion inhibitor 0.1 to 20% by weight, preferably 0.5 to 5% by weight (based on the solids content of the coating composition) of an amide, imide or nitrile of an aliphatic or cycloaliphatic mono-, di-, tri- or tetra-carboxylic acid which is substituted in its (cyclo)aliphatic radical by one or more groups of the formula I

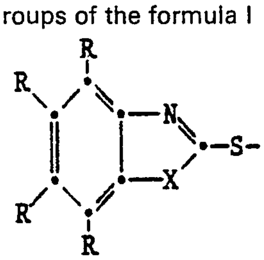

I

in which X is oxygen, sulphur or NH and X is as defined in claim 1, or of a non-toxic base addition salt of such a compound, insofar as it contains a carboxyl group.

13. Coating composition according to claim 12 on a non-aqueous basis, which contains as binder an epoxy resin, polyurethane, aminoplastic, acrylic resin, polyester resin, alkyd resin, phenolic resin, vinyl resin, chlorinated resin, styrene copolymer, cellulose ester, dry oil or a mixture of such resins.

14. Coating composition according to claim 12, on an aqueous basis, which contains as binder an alkyd resin, a polyester, an acrylic resin, polyurethane, epoxy resin, phenolic resin, amino resin, vinyl resin, styrene resin or a mixture of such resins.

15. Coating composition according to claim 12 containing, apart from the binder and the corrosion inhibitor, a pigment a filler or other customary additives for coating compositions.

16. Coating composition according to claim 15 containing a basic filler or a basic pigment.

17. Use of an anticorrosive coating composition according to claim 12 as primer on metallic substrates.

18. Use according to claim 17 as cathodic electroprimer.

19. Anticorrosive aqueous system, outside of the use as coating composition, containing as corrosion inhibitor 0.1 to 50,000 ppm, preferably 1 to 500 ppm, of an amide, imide or nitrile of an aliphatic or cycloaliphatic mono-, di-, tri- or tetra-carboxylic acid which is substituted in its (cyclo)aliphatic radical by a group of the formula I

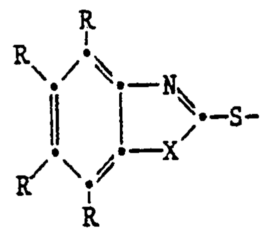

I

in which R and X are as defined in claim 1, or of a non-toxic base addition salt of such a compound which contains a carboxyl group.

20. Cooling water system, air-conditioning system, steam generator, sea water evaporator, hot water system, antifreeze composition, hydraulic fluid, aqueous metal-cleaning or metal-working composition according to claim 19.

21. Aqueous system according to claim 20 containing, in addition to the corrosion inhibitor defined in claim 16, one or more other corrosion inhibitors, complexing agents, reducing agents, antifoams, biocides, emulsifiers, wetting agents or extreme pressure additives.

22. Compounds of the formula II

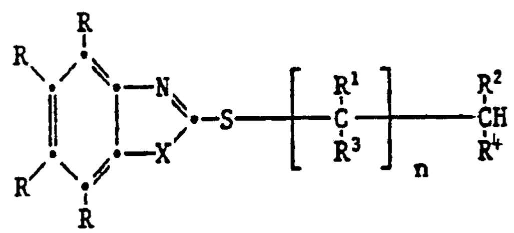

II

in which X is sulphur and each R independently of one another is hydrogen, alkyl, haloalkyl, alkoxy, alkylthio, alkylsulphonyl, cycloalkyl, phenyl, alkylphenyl, phenylalkyl, halogen, —CN, —$NO_2$, —COOH, —COOalkyl, —OH or a primary, secondary or tertiary amino or carbamoyl group, n is 0 or 1 and $R^1$, $R^2$, $R^3$ and $R^4$ independently of one another are hydrogen, alkyl, hydroxyalkyl, haloalkyl, cyanoalkyl, alkoxyalkyl, or phenyl or phenylalkyl each of which is unsubstituted or substituted in the phenyl nucleus by halogen, nitro, alkyl, alkoxy, carboxyl or hydroxyl, or a —COOH, —CN or —$CONZ_1Z_2$ group or alkyl substituted by 1, 2 or 3 —COOH, —CN or —$CONZ_1Z_2$ groups, $Z_1$ and $Z_2$ being independently of one another hydrogen or $C_1$—$C_{18}$alkyl which may be interrupted by one or more O or S atoms or NR° groups (R° = $C_1$—$C_{18}$alkyl, $C_5$—$C_8$cycloalkyl, phenyl, naphthyl, $C_7$—$C_9$phenylalkyl or $C_7$—$C_{18}$alkylphenyl) or may be substituted by —SH, —$NH_2$, —COOH, —COOR°, —$CONH_2$, —CN or halogen, or are $C_2$—$C_{10}$hydroxyalkyl which may be interrupted by one or more NR° groups or O atoms, or are $C_2$—$C_{18}$alkenyl or $C_3$—$C_{12}$cycloalkyl which may be substituted by $C_1$—$C_4$alkyl, —OH, —SH, —COOR°, —$CONH_2$, —CN or halogen, or are $C_7$—$C_9$phenylalkyl, $C_7$—$C_{18}$alkylphenyl or $C_6$—$C_{10}$aryl which may be substituted by $C_1$—$C_{12}$alkoxy, $C_1$—$C_{12}$alkylthio, —COOH, —OH, $NO_2$ or halogen, or $Z_1$ and $Z_2$ together are optionally branched $C_3$—$C_7$alkylene, which may be interrupted by O, S or NR° and which contain 2, 3 or 4 amide groups, —$CONZ_1Z_2$ in the radical

$$—[C(R^1)(R^3)—]_n CH(R^2)(R^4)$$

23. Compounds of the formula II

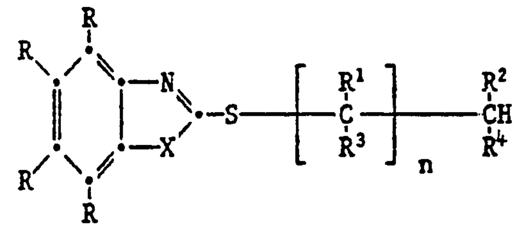

II

in which X, R, $R^1$, $R^2$, $R^3$ and $R^4$ are as defined in claim 22 and which contain at least one $CONZ_1Z_2$ group and at least one —COOH group in the radical

$$-[C(R^1)(R^3)-]_n CH(R^2)(R^4)$$

24. Compounds of the formula II

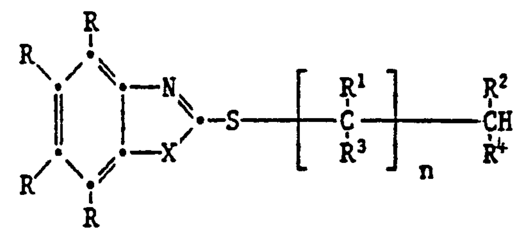

II

in which X, R, $R^1$, $R^2$, $R^3$ and $R^4$ are as defined in claim 22 and which contain one or two groups of the formula III or IIIa

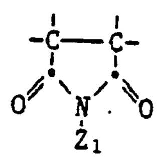

III

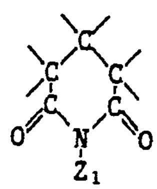

IIIa

25. Compounds of the formula II

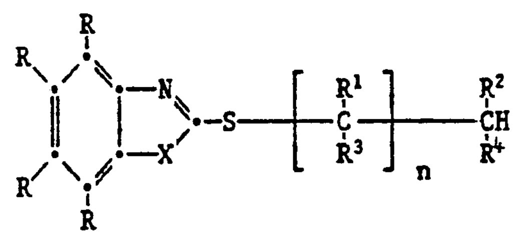

II

in which X, R, $R^1$, $R^2$, $R^3$ and $R^4$ are as defined in claim 22 and which contain 2, 3 or 4 cyano groups in the radical.